# FASCICULE DE BREVET EUROPEEN

(11) **EP 2 791 355 B1**
(45) Date de publication et mention de la délivrance du brevet: **31.05.2017**
(21) Numéro de dépôt: 12810328.0
(22) Date de dépôt: 03.12.2012
(51) Int. Cl.: C12Q 1/68

(54) **UTILISATION DU GÈNE SLC45A2 POUR L'IDENTIFICATION D'UNE POPULATION DE PALMIPÈDES**
VERWENDUNG DES GENS SLC45A2 ZUM NACHWEIS EINER POPULATION VON PALMIPEDEN
USE OF THE SLC45A2 GENE FOR IDENTIFYING A POPULATION OF PALMIPEDS

(30) Priorité: 12.12.2011 FR 1161479
(43) Date de publication de la demande: 22.10.2014
(73) Titulaire: Gourmaud Selection, 85260 Saint-Andre-Treize-Voies (FR)
(72) Inventeur: GOURMAUD, Benoît, F-44100 Nantes (FR); ALLETRU, Bernard, F-85600 Montaigu (FR); JEAN, Christian, F-69500 Bron (FR); PAIN, Bertrand, F-63830 Nohanent (FR)
(74) Mandataire: Godineau, Valérie
(86) Numéro de dépôt international: PCT/FR2012/052779
(87) Numéro de publication internationale: WO 2013/088023

(56) Documents cités:
- FR-A1- 2 921 542
- GRAF JUSTIN ET AL: "Promoter polymorphisms in the MATP (SLC45A2) gene are associated with normal human skin color variation", HUMAN MUTATION, vol. 28, no. 7, juillet 2007 (2007-07), pages 710-717, XP002677313, ISSN: 1059-7794
- TSUBOI KIE ET AL: "Oculocutaneous Albinism in Suncus murinus: Establishment of a Strain and Identification of Its Responsible Gene", EXPERIMENTAL ANIMALS (TOKYO), vol. 58, no. 1, janvier 2009 (2009-01), pages 31-40, XP002677312, ISSN: 1341-1357
- GUNNARSSON ULRIKA ET AL: "Mutations in SLC45A2 cause plumage color variation in chicken and Japanese quail.", GENETICS FEB 2007 LNKD- PUBMED:17151254, vol. 175, no. 2, février 2007 (2007-02), pages 867-877, XP002677314, ISSN: 0016-6731 cité dans la demande

### SEQUENCE LISTING

<110> SAS AGOGENE
<120> Utilisation du gène SLC45A2 pour l'identification d'une population de palmipèdes
<130> PP0000150
<150> FR11-61479
   <151> 2011-12-12
<160> 63
<170> PatentIn version 3.5
<210> 1
   <211> 1567
   <212> DNA
   <213> artificial sequence
<220>
   <223> cDNA du gène ALDH1 du canard de Pékin Anas platyrhynchos
<400> 1
   tgtttcagga acgtcctcaa actcctgatt tgacaatatg aagaaacaag gctcagacag      60
   caatccctct ccagtttttc cagcactgcc agaaccactc aaagatctga aaattaagta     120
   caccaagata tttataaaca atgaatggca caattcagtc agtggcaaaa aatttgaagt     180
   ttttaaccct gcaaatgaag agaaaatctg tgagattgaa gaaggtgaca aggcagatgt     240
   agacaaggct gttaaagcag ctagaacagc ttttgagctt ggatcaccct ggcgtacaat     300
   ggatgcttca gagcgaggaa ggctcttgaa taaactagct gatttagttg aaagagatag     360
   attgatttta gctacaatgg aagccattga tggtgggaaa ctcttttcta ctgcctacct     420
   aatggattta ggtgcctgtg ttaaaacatt acgttactgt gcaggctggg ctgataaaat     480
   ccatggtcgg actgttccaa tggatgggaa ctttttcaca tttacaagac atgagcccat     540
   tggtgtgtgt ggccaaatta ttccttggaa cttcccattg gttatgttta tctggaagat     600
   cgcccctgcc ctttgttgtg gaaacacagt ggttgtcaaa ccagcagaac aaacaccact     660
   gactgccctt tacatgggat ccttaattaa agaggcagga tttccacctg gagttgtgaa     720
   cattgtgcca ggctttggac ctactgctgg agctgctatt tctcaccaca tggatataga     780
   taaagtggcc ttcacaggct ctactgaggt tggcaaactg atcaaagaag ctgcaggaaa     840
   gagcaacctg aaaagagtta cattggaact tggaggaaaa agtcctaaca ttatatttgc     900
   agatgcagat ttggacacgg ctgtggaatt tgcacatatt ggtctattct accaccaagg     960
   acagtgttgt atagcaggat ctagaatttt tgtgaaagaa aaccaattca ctatgttgtg    1020
   tcatagagct tccttgaaag ccaaaataaa tatccccaca aatagaagta gtgtttgcac    1080
   aaaagtcccc ccagaaattg ataaggaaca gtttcaaaaa atcctggagc taattgagag    1140
   tgggaaaaaa gaaggagcca aactggaatg tggaggaggt ccatggggag acaaaggtta    1200
   cttcatccag cccacagttt tttctaatgt tacagatgat atgcgcatcg ccaaagaaga    1260
   gatatttgga cctgttcaac aaattatgaa gtttaaaact gtagatgaag ttatcaagag    1320
   ggcaaataat actagttatg gcttagcagc agcagttttt accaaagaca ttgataaagc    1380
   actgacattt gcagctgctc ttcaagctgg gacagtatgg gttaattgtt atagtgcaat    1440
   gtctgctcag tgtccttttg gaggatttaa gatgtcagga aatggacgag aaatgggaga    1500
   atacggcctt catgaataca cagaagttaa gactgtcact atcaaaatcc cacagaagaa    1560
   ctcataa                                                              1567
<210> 2
   <211> 1524
   <212> DNA
   <213> artificial sequence
<220>
   <223> cDNA du gène CECR2 du canard Pékin Anas platyrhynchos
<400> 2
   cagaggccag atgtcttgac cactggagct gggaacccaa ttggggataa gctcaatctt      60
   atgacagtag ggccccgtgg tcctcttctg gttcaagatg ttgttttcac tgatgagatg     120
   gctcattttg acagagagag gattcctgaa agagttgtgc atgcaaaagg agcaggtgct     180
   tttggctatt ttgaagtgac tcatgatatt acgaagtact gtaaggcaaa agtgtttgaa     240
   cacattggaa aaagaacacc aattgctata cggttctcaa ctgttgctgg agaatctggg     300
   tcagctgata cagttcgtga ccctcgaggg tttgcaatga aattttatac agaagagggt     360
   aactgggatc ttgtgggaaa caatactcct atcttcttta ttcgggatgc aatgttgttt     420
   ccatccttca tccatagcca aaagaggaac cctcagactc atttgaagga tgcagatatg     480
   gtatgggact tttggagtct tcgccctgag tctttgcatc aggtgtcttt cttgtttagt     540
   gaccgtggta ttcctgatgg atatcgccat atgaatggat atggatcaca tacttttaaa     600
   ctgattaatg ctagtgggaa agcagtttat tgcaagtttc atgtgaagac tgatcagggc     660
   atcaaaaatc tttctgttga ggaagcagga aggctggctt ctactgatcc tgattatgga     720
   atacgtgatc tttacaatgc catagcccat ggcaactacc catcctggtc tttctacatt     780
   caagttatga catttgaaga agcagagaag tttccattta atccttttga tttaactaag     840
   atctggccac atggtgacta ccctctcatt cctgtgggaa agcttgtttt gaacaggaat     900
   cctgtcaatt actttgcaga ggtagaacag atggcatatg atccaaacaa catgccacct     960
   ggtattgaac ctagccctga taaaatgctg cagggtcgtc tttttgcgta tgctgataca    1020
   catagacatc gtctgggacc taactaccta caaattcctg tgaattgccc cttcagagct    1080
   cgtgtagcca attatcagag ggatggacca atgtgcgtga ctgacaacca aggtggtgcc    1140
   ccaaactatt atccaaacag ttttactggt ccagaagatc agcctgttgt gaaggagagc    1200
   cgcatgtgta tttcaggaga tgtgcagcgt ttcaatagtg caaatgaaga taatgtgtct    1260
   caggtgcgag atttctatat caaagtgctg aaggaggatg aacgtcaaag gctgtgtaaa    1320
   aatattgctg atcatcttaa agatgctcaa ctcttcattc agaagagagc tgtgaaaaac    1380
   ttcactgatg ttcatcctga ctatggtgcc cgtattcaat cattgctgga caaatacaat    1440
   gctgaaagtg ggaaaaagga tgtaattaag acgtacacac aagccacctc tcgcatgtct    1500
   gccaaagaaa gatccaactt gtaa                                           1524
<210> 3
   <211> 859
   <212> DNA
   <213> artificial sequence
<220>
   <223> cDNA du gène EDN3 du canard Pékin Anas platyrhynchos
<400> 3
   aggaggactg ttccatatgg gctgtctaac tacagaggca gttttagagg caaaagatct      60
   acaggccaga atcaaagtac tttccagtct tcttttcggt gttcctgttt ggatactgat     120
   gacaagcagt gtacacagtt ttgcaggaga atgcaagaca ggagaagaaa tcatggatca     180
   atgaaaacaa cagaggaaaa agaccaaagc agggaagagg agaacgcttt cattcagtag     240
   cacagagagc ttggaggatc ctgaacagtc aggtatggct cagctttaac attcatttgc     300
   tgaagctcag aagcaagaac ttaaactaca gctctctcct gagatggaca acattgatgc     360
   ttcagtctga tgccagtgga gaattcagca gcatgcaaca cttccattcc ctcaatgcca     420
   ttctgagggc acgtgaacct gtatgaagaa gctttctccc atgcttcccc cgtgagagtc     480
   tcagtaaagg ataattatcg cataacctgg cagggaatat atatatcaat atttgaacac     540
   aaatacaaag actgattcaa acttcagcaa cacaatttca ggaaacattt ttctacctga     600
   tttttaaaag taaaatagaa acatactgtt tcacttatgc ttgctatgtg tctgaagaac     660
   ccatccagtt ttacgtgggc aattgtaact ggacaacgtg atgagttaga gagagatcgc     720
   ctatacatga gttctcaata agaataaaca gattcaaaat gtttctcttg ttaatgtatg     780
   caagcctgaa tcatcatatt gtaaccttgt ggcagatgtt taaataacaa tatctatgca     840
   gaataaatat gaacggcta                                                  859
<210> 4
   <211> 1297
   <212> DNA
   <213> artificial sequence
<220>
   <223> cDNA du gène EDNRB du canard Pekin Anas platyrhynchos
<400> 4
   cccaagggtt ttgggggggt gggcggcggg cggccccgct cggcctcgcc ccccatgtgc     60
   acggggcaga cggagatcaa ggagactttc aagtacatca acacggtggt gtcctgcctg    120
   gtcttcgtcc tgggcatcat cggcaactcc acgcttctgc ggatcatcta caagaacaag    180
   tgcatgagga acggccccaa catcctcatc gccagcctgg ccctgggcga cctgctgcac    240
   atcatcatcg acatccccat caacgtctac aagctacttg cagaggactg gccctttggt    300
   gttgaaatgt gtaaattagt gcccttcatt caaaaggcat cagtgggcat cacagtgttg    360
   agtttgtgtg ccctcagtat agacagcagg taccgagcag tagcttcttg gagtcgaatt    420
   aaaggaattg gagtgccaaa gtggactgcc gttgaaattg tactgatctg ggttatatca    480
   gtggtattag ctgttccgga agcaattgca tttgatatga ttacgatgga atacagagga    540
   aaggatctca gaatctgcct acttcacccc acacaaaaaa catcctttat gatgttttac    600
   aagcaagcta aagactggtg gttgttcagc ttttacttct gtttgccact ggctatcaca    660
   gcacttttct acactctcat gacctgtgaa atgttacgaa agaaaagtgg gatgcagatt    720
   gctttaaatg atcacttaaa acagagacgt gaggtggcca aaactgtgtt ctgcctggta    780
   cttgtttttg ctttgtgttg gctccctctt cacttaagca gaatattgaa actcactatt    840
   tatgatcaaa aggacccaaa tagatgtgaa cttttaagct tttttcttgt gatggattac    900
   atcggcatta acatggcttc actgaattcc tgcatcaatc caattgctct gtatttggtg    960
   agcaagagat tccaaaactg ctttaagtca tgtttgtgtt gctggtgcca gtccaaagat   1020
   ctgttgtccc tggaggaaag gcagtcctgt ttaaagttca aagctaatga ccacggatat   1080
   gataacttcc gctccagtaa caagtacagc tcttcataaa aacaggcata aaaggtatat   1140
   tctcttacat taatgctggt gcctttttaa tgaaatggta acatttagaa aactctagtt   1200
   ttgtgtttgc acaaaaacaa aagaatatca ttttaaaaaa aaaaaagtga aatctgtgca   1260
   actctcaaag tcatggacat ttacaaaact ctaatct                             1297
<210> 5
   <211> 1300
   <212> DNA
   <213> artificial sequence
<220>
   <223> cDNA du gène GPR143 du canard Pékin Anas platyrhynchos
<400> 5
   gcaacagcag ctcctgctcg tgggccccct ccaccagccg gacgcttcat ggcttctccc       60
   cgcttagaaa cctactgctg cccgaaccgg gatgcagcca cgcagctcgt gatgagcttc      120
   cagcccgagg tcttctgcgg ggtctgcatt ggcagcgcct ccgtcagcgt gctgctgacc      180
   gtcctgcagc tcctgccaaa gaagggacag atcctgcgga agatgcccaa agcctcctcc      240
   tcctccacca tccttctcct tatctctgtt tgtgacatcc tcggtggttt aggtgtgatc      300
   ttcagatcca gcgtctggtt gggctttcca ggcttcatcg ccaacatctc ggtggcgaac      360
   gtgaccgaca tgtggccctc tgccttctgc gtggggagcg cgatgtggat ccagctgtta      420
   tatagtgcgg gcttctggtg gttgttttgc tatgctgtcg attcttactt ggtggtaaga      480
   agatcagcag gactgagtac gatcgtgctg taccacatga tggcctgggg cctggcggtc      540
   ctgctctgcg tggagggagt ggcgctgctt tactacccgt ccctgtccag ttgtgaaaaa      600
   ggcctggagc atgcaattcc ccattacatc acaacctacg ccccactcct gctggtgctg      660
   gtggtcaacc cgatcctgtt tagaaggaca gtatctgcag ttgcctcttt actgaaaggc      720
   agacaaggga tttacacgga gaatgagaga cgtctgggga cagagatcca gatgcgcttt      780
   ttcaaaatta tgctggtatt cattgtttgc aatagttggt catctaatat cgtcaatgag      840
   agccttttgt tctatcttga gatgcagcca gatatcaatg aaatgcctct gaaaaacatc      900
   agaagcgcca cactgatcac atggattatc atggggattc ttaatcccat gcaaggcttc      960
   ctcttcacat tggctttcta tggttggacg ggatgcagag tggacctgaa atggcagaag     1020
   agagaaatac cctgggaatc catgtcctcg tcaaccgtgg gtgaaaatgc ctatccctca     1080
   cctgtgaact accaaaccaa cctccacgat ccaaagaagt tatcgacaac tgacagccag     1140
   cagactgatg aggctataag catgttgtct gaaggtaaca ccagtggtga tgacaggata     1200
   accaggagct ctcccatcta ccagggctgg tagttcatag gtgcagagct gaatctcact     1260
   tctcccactg tcaagactcg taaaaccaca tcactgtgtg                           1300
<210> 6
   <211> 4863
   <212> DNA
   <213> artificial sequence
<220>
   <223> cDNA du gène LRP6 du canard Pékin Anas platyrhynchos
<400> 6
   tttggctctg aaattagggc tttgagtcag gtgtcattat gtattatatt tgaagctgcc       60
   ccgctgttgc tgtatgcaaa ccgacgtgac ctgcgcttgg tggatgctgc acatgggaag      120
   gagaacgcca ctgtgatcgt gggtggccta gaggacgcgg cagcagtgga cttcgtgttt      180
   ctgaaggggc tgatatactg gagtgatgta agtgaagaag ccattaaacg aacagaattc      240
   aataagtctg ggagtgtaca aaatgtggtc atctctggac ttttgtcacc tgatgggctg      300
   gcatgtgact ggctgggaga gaaattatac tggacagatt ctgaaacaaa tcggattgaa      360
   gtttccaatt tagacggatc actgaggaaa gttttgtttt ggcaagaatt ggatcagccc      420
   agagcaattg ccttagatcc tgcaagaggg ttcatgtact ggacagactg gggagaagtg      480
   ccaaagatag aacgtgctgg gatggatggt tcaagccgct ccattatagt caacacagac 540
   atttattggc caaatggatt gacgttggat tatgaggaac agaaacttta ttgggcagat 600
   gctaaactga attttatcca caaatcaaat ctcgatggaa gtcatcggca agcggtggtt 660
   aaaggctccc ttccacatcc ttttgctttg acgctgtttg gggacacgtt gtactggacg 720
   gactggaaca cacattcaat cttggcctgc agcaagtact ctggggagga cctgcgtgaa 780
   atacattcca acatcttctc tcccatggat atccatgcgt tcagccaaaa gaggcagcca 840
   aatgctacaa acccatgtgg aattaataat ggtggctgct cccacttgtg tttgatgtct 900
   cctaccaagc cctcttatca gtgtgcatgt cccactggtg tgaaacttct tgagaatgga 960
   aagacctgca aagatggtgc cactgaattg ctgcttctag cccgccggac agatttgaga 1020
   cggatttcat tagacacccc agattttact gacattgttt tgccactgga ggacatccgt 1080
   catgccatcg ccattgattt tgatcctctg gaagggtaca tctattggac tgacgatgaa 1140
   gttagggcca ttcgccgctc atttatcgat gggtcaggta gccagtttgt tgtcacagca 1200
   cagattgctc atcctgatgg cattgctgtg gactgggttg ctcgaaatct gtattggact 1260
   gacactggca cagaccgtat tgaagttaca aggcttaatg ggaccatgag aaaaatcttg 1320
   atatcagaag acctggaaga acccagagct attgtgctgg atcctatggt tgggtacatg 1380
   tactggactg actggggaga aatcccaaag atcgagaggg cagcattaga tggctcagac 1440
   aggattgtgc tggttaacac ctcacttggc tggccaaatg gactggcact ggattatgaa 1500
   gaaagcaaaa tatactgggg agatgccaaa acagacaaaa tagaggtcat gaatgctgat 1560
   ggaactggga gaagagttct ggtggaggac aagctgcctc atatctttgg attcacgttg 1620
   ctgggagact atgtttattg gacagactgg caaagacgca gtattgaacg tgtacaaaaa 1680
   cgaacagcag aaagagagat catcatagat caactgcctg atctaatggg cctcaaagct 1740
   acaaatgtcc gccagataat tggcttaggt acaaacccat gtgcagagga taacggcggt 1800
   tgcagccatc tctgtctgta cagaccacaa ggccttcgct gcgcttgccc gataggcttg 1860
   gagctcatca atgatatgaa gacctgcatt gtcccagagg ctttcctgct gttttctcgg 1920
   agagcagata tcagacggat ctctttagaa acgaacaaca acaatgtcgc tattccactc 1980
   accggagtta aggaagcttc tgctctagat tttgatgtga cagataatcg aatttactgg 2040
   actgacattt cattgaagac catcagcaga gcatttatga atggcagcgc actggaacat 2100
   gttgtagagt ttggcttgga ttaccctgaa ggcatggctg tagattggct ggggaagaac 2160
   ttgtattggg ctgatactgg aacaaatcgc atagaagtgt caaagctgga tggacagcat 2220
   cgacaagtgt tggtgtggaa agatctcgac agtccacggg cactggcgtt ggaccctgct 2280
   gaggggttta tgtactggac tgaatggggt ggtaagccga agattgatag agctgctatg 2340
   gatggcagtg agcggactac tcttgtaccg aatgtgggac gtgctaacgg cctaactatt 2400
   gattacgcta aaagacgact gtactggacc gaccttgata ccaacctgat agaatcctcc     2460
   aacatgctgg aaacaggcct tgaccgtgag attatagctg atgacctgcc tcacccattt     2520
   ggcctgactc agtatcagga ttacatttac tggacagact ggagccggcg tagcattgaa     2580
   cgagccaaca agaccagtgg ccagaacaga actatcatcc aagggcattt ggattatgtt     2640
   atggacatct tggtcttcca ttcctccagg caggcaggct ggaatgagtg tgcctctagt     2700
   aatggtcact gctctcatct ctgcttagca gtccctgttg gtggttttgt ctgtgggtgc     2760
   ccagctcact actccttgaa cgctgacaac aggacttgta gcgtccatca agctcctaca     2820
   acctttcttt tgttcagtca gaagaatgca attaatcgca tggtgataga tgaacagcag     2880
   agcccagata tcatactccc tatacatagt ctcagaaatg ttcgagccat cgattacgac     2940
   cccctggata aacagctgta ttggattgat tctcggcaga acatcattcg gaaggcacag     3000
   gaagatggca gccagagcct cactgttgtg acaagcccag ttcccaacca gaacttagac     3060
   atgcaacctt atgacctgag cattgacatc tacagccgct atatctactg gacctgtgaa     3120
   gctactaatg tgatcaatgt gacacgcctg gacgggagat ctatgggagt ggtgctcaaa     3180
   ggagatcaag accggcccag agccattgtg gtgaatccag agaaaggata catgtatttc     3240
   accaacctac aggaaaggtc tcctaaaatt gagagagcag cattggatgg aactgaacgg     3300
   gaggtccttt ttttcagtgg tttgagcaag cccatagcct tagctattga cagccagcta     3360
   ggcaagttgt tctgggctga ttcagacctg cgaagaattg aaagcagtga cctttcagca     3420
   ggtgctaacc gaattgtttt ggaagactct aatatcttgc aacctgtggg actaactgta     3480
   tttgaaaact ggctgtattg gattgacagg caacagcaga tgattgaaaa gattgatatg     3540
   actggtcgag aaggacgtac aaaggtccaa gctcgtattg cacaacttag tgacattcat     3600
   gctgtgaaag agctcaacat tcaggaatac agacaacacc cttgttctca agataatggt     3660
   ggttgctcac acatttgcat tgtgaagggc gatggcacca cacgctgctc ttgcccagtc     3720
   caccttgttc tgctgcagga tgagctgtcc tgtggagaac cgccaacttg ctcccctcag     3780
   cagttcacct gtttcacagg tgagattgac tgcatcccag tggcctggcg ctgtgatggt     3840
   ttcactgaat gtgaggacca cagtgatgaa aaaaactgcc cggtgtgctc agacacccag     3900
   ttccagtgtg aaagcggaca gtgcatagac agtgccctcc ggtgcaatgg agaagcaaac     3960
   tgccaagaca actcagatga gaaaaactgt gaagtgcttt gtttaactga tcagttccgc     4020
   tgtgccagtg ggcagtgcat tggtaaaagc aagaaatgtg atcacaacct ggactgtagt     4080
   gacagctcag atgaacaagg atgctacacg acagaggagc ctgctccaca gcccaacaac     4140
   acgataggct ccatcattgg tgtgatcctc acagtttttg tggttggagc aatgtacttc     4200
   atctgccaga gggtgctatg cccacgcatg aagggagatg gggaaacaat gaccaatgat     4260
   tatgtggtgc acggaccagc ctctgtacct cttggttatg tgcctcaccc gagctctttg      4320
   tcaggatctc ttcctgggat gtctcgaggt aaatctgtga tcagctccct cagcattatg      4380
   ggagggagca gtggaccacc ctatgacagg gcccatgtca ctggagcctc ttccagtagt      4440
   tcttcaagta ccaaaggcac ttactttcct ccaattttga acccaccacc ttcaccagct      4500
   actgaacgtt cacattatac catggaattt ggttattctt caaacagccc atccactcat      4560
   agatcctaca gctacaggcc ttacagctac cggcattttg cacctcccac gaccccctgc      4620
   agcacggatg tctgcgacag tgactatgcc cccagccgaa gggtgacagc agcaacggcc      4680
   aagggctaca ccagtgactt gaactatgat tcggagcccg tcccgccacc tccgactccg      4740
   cgcagccagt acctgtcagc ggaggagaac tatgagagct gcccaccctc cccgtacacg      4800
   gagcggagct actcgcacca tctctacccg ccaccgccat ccccctgcac agactcctca      4860
   tga                                                                    4863
<210> 7
   <211> 2076
   <212> DNA
   <213> artificial sequence
<220>
   <223> cDNA du Gène MLPH du canard Pékin Anas platyrhynchos
<400> 7
   atggggagga agctggatct ctccaagctg accgatgagg aggccaggca cgtctgggag      60
   gtggttcgga gggacttcga cctgcggaag aaagaggagg aacggctgga ggagctgaag     120
   tgcaagatcg accaggaaag cagcaagagg gcgttcctga caaatcagtc ccacctcaac     180
   gaaacccact gcgtccactg cctgcagccc ttcaagttcc tgctgaacag caagcggcag     240
   tgcctggact gccacttcta cacctgcaag agctgcagcc gctacaacaa gaaggagcag     300
   ggctgggtct gcgacccctg ccgcctctcc aggattgtga agatcgggtc cctggagtgg     360
   tactacgagc acgtgaggtc ccggttcaag aggtttggaa gcgccaaggt gctgcagtcc     420
   ctctacggca ggctgcagcc agggcagcgg gacaactcgg catttctagg tcttcatgac     480
   agagtttaca gcctgccaga cataaacagt gaatgccagc tccgtgccgg cggcagcatc     540
   ggggatgaca gtgatgatga agacgatgcc cttcgtgggg ccgaagcaga gtgttacagc     600
   agggctaaga tgcgcaagac aaagcgcctg ctgtctgtcc atccctttga tttcgaagtg     660
   gactcggagt actcttccca gtctcgccgc cagtctgtgc agctttctcc agcaccaatc     720
   agcccggatg gcttccagtc cttcccaggc ttccccagct cggctgaaga tgccccccag     780
   gagcacagga tggaggaagc aggtctggca gcagtcttcc accacatctt gcaagaacag     840
   gaacagcacc caagccctcc agagcagctc ttcagcacag aggttcatct caccatcaat     900
   tcgcggagaa ggagcctgga aagaaacaca aagcctggca gcccctgggg tgaacagccc     960
   cggtccccat actctgcaga catggacact tctgacgaag atgtgaacgg agcccagaag 1020
   gtccctgcct acctgcccca ccacgtgaaa cgccggagca gagcctcgtc ccaggagaac 1080
   atcagccact ctggaaacca gattcatgag ctcaacaaac gcatgtctgc aatcgaacgc 1140
   atgctgaacc gcttggagga aaaaatcctg gtgcactctg ccgagtctcc ggccccagag 1200
   ccccagctcg atcctgacgc cgaggaggag gagctgaaga ggaagctgca ggagctagcc 1260
   agccacatca gcgacaaaga agtttcctct gaagaagagg agcgtgaaga aaagacgcga 1320
   gtgaagaaac cagagatgat cttctccagc gatgacatgg ccagtgaggc tcgaaagagg 1380
   tcgtcggctc aggctttgag cgaaatcacg actgaagtcc tgagagccat aaacgccacg 1440
   gagaaagcgg tctgtgagtc ggtgcatgga gaaagccagc gcaacgggcc ctgtgccccc 1500
   ctcggagggc agcatcccag cctggaaggt gggaaggagg tggccgaagc gtaccgtgag 1560
   ctggaagaaa atgtgtacct gactgctggg aagacgtacc agctcgagac aacccttgtg 1620
   gagcttgagg aaggggctca gcacagcggc acgaccgact cagagctgtc ggagctggag 1680
   gacaaagtgg cctcggcagc tgctcgggtg cagcaggcag agagcgagat atcggacatc 1740
   gagactcgaa tcgcggccct gtccgcggca gggctgaccg tgaagtcggt ggaaaaggca 1800
   aggaagagcg ggcagtggtg gaaaggcagc ctgtggggtg cctcggtccc cctgtcccct 1860
   tccccaccac tgacatctcc ccctctcttg caggagatgt ccggactgca gttcttcagg 1920
   aggaaattcc acagccccct cgatatcagc agtcccgacg actccttcga ccgcaactca 1980
   gtgtaccgcg gctcgctgac gcagaggaac cccaacggga agagcaggag ggcagagcgg 2040
   ctctttgcga agcccgtgat gacccacctg ccctga 2076
<210> 8
   <211> 2202
   <212> DNA
   <213> artificial sequence
<220>
   <223> cDNA du gène OCA2 du canard pékin Anas platyrhynchos
<400> 8
   aggtatcact tcaatttctc caaattattc agctcaaggt caagaaacac ttgctttaca        60
   gagaagacac cactgctgaa ggtctcctca gaggacaatg ggttgctgtg catggctatt       120
   cacaatccag attttaccac agatgatgat tcatgggaca acagctctgc agaatttgag       180
   caaagatttc aactgggaag tgaaatgacc agtttgtcta gatctgcttc ttctgagaaa       240
   tatgaaatct tggacaacct tcatgccaag ttcaatttat caaagatgag gtgctgtcta       300
   aaattcctga aagtttcagg tctttttatc tttgtagttg tctgctctgt tttatttggc       360
   atttatccag atcaaggtat gtcctggcag atgttggctg tctcgcctct ggaaagcttc       420
   tctatgaatc tgacagattt tcatgattct gctctgttga ggttagacct tggaggtcca 480
   tttgtagcag atgtagagga tgagcaaaca gaggaataca ttttagtgca aatcagccag 540
   actgaagatg ctgtcccaag gaggagacac cagcagcagg tggtgtacaa ctggtctcta 600
   cctttaagtt caagaagaaa tcagcaaatc atcacaacta gaacctttca gatgccaaac 660
   agaggcacca tcttcataaa cattcaagct ttcctgcagg agcctggaag cgttcctctc 720
   tccatgaaac atcaatacct tcatgctaat atagaggcac aagtaacaat tgcatccatc 780
   atcttagttg gtgtctatgt gctgatcata ctggaaattg ttcacaggac ccttgcagcg 840
   atgctgggtt ctttggcagc tttagctgca ttagctgctg tgggggagag gccaagtatg 900
   gtcaaagtgg tagagtggat tgattatgag acactggcgc tactgtttgg aatggtggtt 960
   ttggtggcca tattttcaga aactggattc tttgactact gtgcggtaaa ggcttatcga 1020
   ttctctagag gcaaggtgtg ggccatgatt acacttctgt gccttattgc tgcaattctt 1080
   tctgcatttt tggacaacgt taccactatg ctgctcttta cgccagtaac cataaggctc 1140
   tgcgaagtac ttaatcttga tcccaggcat gtcctgattg cagaagtaat tttcacaaat 1200
   attggggggg ctgccacagc tgttggggat cctccaaatg tgattattgt ttcaaagcag 1260
   gagctgagga aggagggatt ggattttgca gccttcacag gacatatgtt tgttggtatc 1320
   tgccttgttg ttctggtctc ctttcctttt ctcagacttc tttactggaa caaaaaactt 1380
   tacaataagg agccgagtga aattgttgag ctgaaacatg agatctatgt ttggaggttg 1440
   actgcacagc gcattaaccc tgccagcagg gaagagactg ctgtaaaatg cctcttgatg 1500
   cagaaggtgc tgactctgga gatgctgctg aggaagaagc ttaggacgtt ccacaagcaa 1560
   atttcacaag aagataaaaa ctgggaaaca aatattcagg aacttcagaa aaaacacaga 1620
   ataacagaca aaatccttct tatcaaatgc ctgactgtgc tgggatgtgt aattcttatg 1680
   ttttttctca actcatttgt tcccggtgtc tacctagatc ttggctggat tgccattttg 1740
   ggagccattt ggctgctagt gctagcagac atccatgact ttgagatgat attaaaccga 1800
   gtggaatggg caacccttct tttctttgca gcattgtttg ttctcatgga ggctttggct 1860
   catttgcatt taatagacta cataggggag cagacagctt tgttaataaa ggttgttcct 1920
   gaagaacagc gcttggcggt agccatcatt ttagtgctct gggtgtctgc tttggcctcg 1980
   tccgtgattg acaatattcc gttcacagct acgatgattc ctgtgcttct caacctgagt 2040
   aaagatcctg atgttaactt gcctatgaag ccattaatct tctcgttggc catgggtgca 2100
   tgccttggag gtaacgggac attgattgga gcatctgcaa atgttgtttg tgcaggaatt 2160
   gctgaacagc atggttacgg cttctctttc atggagtttt tc 2202
<210> 9
   <211> 1499
   <212> DNA
   <213> artificial sequence
<220>
   <223> cDNA du gène PAX6 du canard Pékin Anas platyrhynchos
<400> 9
   acccatgcag atgcaaaagt ccaagtgctg gacaatcaaa acgtgtcgaa tggatgtgtg     60
   agtaaaattt tgggcaggta ttacgaaact ggctccatca gacccagggc aatcggaggt    120
   agtaaaccga gagtagcgac tccagaagtt gtaagcaaaa tagcgcagta taaacgagag    180
   tgcccctcca tctttgcgtg ggagattcga gacagattac tatcagaggg ggtctgtacc    240
   aacgataata tacccagtgt gtcgtcgata aacagagtcc tacgcaacct ggctagcgaa    300
   aagcaacaga tgggtgccga cgggatgtac gacaagctaa ggatgctgaa cgggcagacg    360
   gggacctggg gcacccggcc cggctggtac cccggcaccg gatttccttc tctcctttcc    420
   gcagacggct gtccgcagca ggagggagga ggggagaaca cgaactccat cagctccaat    480
   ggggaagatt cagatgaggc ccagatgaga ctccagctga aaagaaagct gcagagaaat    540
   aggacatcct ttacccaaga gcaaatcgaa gcccttgaga aagaatttga gaggacccac    600
   taccctgatg tgtttgcaag agagagacta gctgctaaaa tagacctgcc tgaagcaaga    660
   atacaggtgt ggttttctaa cagaagggcc aaatggagaa gggaagagaa gctgaggaac    720
   cagcgaagac aagccagcaa cactcccagc cacattccca tcagcagtag tttcagcaca    780
   agcgtttacc agccgatccc gcagccaacc acccccgttt cctctttcac atcaggttcc    840
   atgttgggca ggacggacac ggcgctcaca aacacgtaca gcgcgctgcc gcccatgccc    900
   agcttcacca tggccaacaa cctgcctatg caacagcccc cggtacccag ccagacctcc    960
   tcctactctt gcatgctgcc caccagcccc tcggtgaacg gccggagcta tgacacctac   1020
   acccccccgc acatgcagac acacatgaac agccagccga tgggcacctc tggcaccact   1080
   tccacaggtc tcatttcccc tggagtgtca gttccagttc aagttcctgg cagtgaacct   1140
   gatatgtctc aatactggcc aagattacag taaaacatgt gttaattctg taaatgacta   1200
   tatggacaac agttggatgt tcagcagtat tttctaaagg aagaatggct ctcagagtac   1260
   ttctgtactg caactgtgcc ctatgctgta acacatggaa aagactttaa catggacctt   1320
   tgtacactga aggcattata tcggttggaa caaatcttca ttttggtatc caaactttta   1380
   ttcattttgg tgtattattt gtaaatggcc atttgtatgt tataatgaaa aaaaaaaaag   1440
   aacaatgtag actggatgga tgtttgatct gtgttggtca tgaagttgtt ttaagaaaa    1499
<210> 10
   <211> 763
   <212> DNA
   <213> artificial sequence
<220>
   <223> cDNA du gène RPS17 du canard Pékin Anas platyrhynchos
<400> 10
   atcatcgaga agtactacac gcggctgggc aacgacttcc acaccaacaa gcgggtgtgc      60
   gaggagatcg ccatcatccc cagcaagaag ctgcgcagcc ccgtgtccca caggtatgtc     120
   acccacctga tgaagcggat ccagaggggc cccgtcagag gcatctccat caaactccag     180
   gaggaggaga gggagaggag ggacaactac gtcccagagg tctctgccct cgatcaggag     240
   atcatcgaag tggacccaga caccaaggaa atgctgaagc ttctggactt tggcagcctg     300
   tccaacctgc aggtcacaca gcccacggtg gggatgaact tcaaaacacc ccgaggagct     360
   ctctgaggcg gtcgctgtta tgtcactttt ccaataaacg tgggaaacca actcggactg     420
   cttggtcttt cgaagcctgt gcgcgcaggg gtgaggaggg gagggcagtt tctctcggga     480
   tatcacatct aaggcttcac tgtgtgaaga ggacttgaca ccttcacatt tgtgagcgcc     540
   ttttgggggt ctttccttgg gcatgaggtg caaagctgtt attttacagg ttttcctgca     600
   ggcatgcgtg cttgctgctc tcaggatggt ggcgcacagc caggagaggg cgagcgtggt     660
   gtgtcctcac catcagcacc tcccagcttt gtgctgtgct gcttgtgagt gggtagctgt     720
   cagggccgtg ctgtgccaga ataaaagttg ccttttataa ccg                       763
<210> 11
   <211> 1617
   <212> DNA
   <213> artificial sequence
<220>
   <223> cDNA du gène SLC45A2 du canard Pékin Anas platyrhynchos
<400> 11
   cagaccaaat ccacccggca tatgacccgc gctccatgtc agccagagca ggaggtggcc      60
   atgaccgacc tggaccacac cagagggaaa gagaaggcta tgcagcaatc cgtgaccagg     120
   accgggatgg tggtaccgaa gaagcgaccg acgggaaggc tggtcatgca cagcatggcg     180
   atgttcggca gggagttctg ctatgctgtg gaggctgtgc tgctcagtgt agggctgccc     240
   aagagcctct acagcctggt gtggctcatc agcccgatcc tgggcttcgt gctgcagccc     300
   gtggtaggtt cagccagtga tcactgcacc tgtagctggg gcaggaggcg accgtacatt     360
   ctgggtctgg gcatcataat gctggtaggc atggctttgt acctcaatgg ggacgagatg     420
   atctcagctt tcatcgatgg gagagacaag cagacgtggg cgatagttgt taccatgctg     480
   ggagtagtgc tttttgattt tgcagctgac tttattgatg gccctatcaa agcatattta     540
   tttgatgtct gctctcatca ggataaagag aagggtctgc attgccatgc cctcttcaca     600
   ggcttaggag gagccctggg ttacctgaca ggtgctgtgg attggggtca aaccgtacta     660
   ggatattcct taacatcgga attccaggtg attttcctct tgtcagcctt ggttttccta     720
   atctgcctta ctgtacatct gcgcagtatt cctgaagtcc cgctcagata taacaaagaa     780
   gagacaaggt tattgttgga agtgactgac cccaggaagt atagctccat agaggaaatc     840
   aggaatggat acatagcatg tactgaaata aatgctgcag caaaaccaaa gaagcacgct     900
   gctacatcat gtacagagac tcaaaggcgg atgaccctta agtcactctt gaagacgctt     960
   ttcagcatgc catcccacta tcgttatctg tgtgtgagcc acctgtttgg atggatggct    1020
   ttcctgtcca acatgctctt cttcacggat ttcatgggac aggttgtata ccgaggtagt    1080
   ccttatgcac ctcataactc cacagtttac cttacctaca gaagaggcgt agaggttgga    1140
   tgctgggggc tgtgcatcaa tgcaatttca tcctcagtct attcttacct gcagaaaatc    1200
   cttctgcaat acataggatt aaagggactt tatttcattg gatacctact ttttggatta    1260
   ggtactggat taattggctt gtttcccaat gtctattcca ctctggctct gtgttccctc    1320
   tttggtgtca tgtccagcac actgtacaca gtgccattcc aactcattgc agagtatcac    1380
   agagaagaag aggtccagag tctgcaggaa gatgaacaag acacagatca caggcgaggg    1440
   aagggcattg actgcgccgc tcttacctgc atggtccagc tggcccagat cattcttggt    1500
   gtgggcctgg ggctcctggt tagtgtagct ggcagtgtgg tcactgtgat ttctgcatcg    1560
   atggtgtcac tgatcggctg ctgctttgtt gctttttgtg ttcgatatgt ggggtag       1617
<210> 12
   <211> 2172
   <212> DNA
   <213> artificial sequence
<220>
   <223> cDNA du gène TBP du canard Pékin Anas platyrhynchos
<400> 12
   ttgtttagcc aatattctca agatactact tcttttcttt gcaggttctg tttagcattt        60
   caggctcgcc acacgcattt ctctggagct gatgtttttg taacgttttc agcttggcta       120
   aaaggctgct ctgctggaat gctttaaagg gttagaaacc ccttcggttt gaatttaatt       180
   tcagtcaatt gagggaaata tttctgatca taccaaagaa ataactggaa acatggatca       240
   gaacaacagc ttgccgccct acgctcaagg cttagcctcc cctcagggtg caatgactcc       300
   cggaattccg atttttagcc cgatgatgcc atatggcaca ggactgacac cgcagcctgt       360
   ccagagcacc aacagcctgt ccatccttga gtctacatca cagcaagcga cacagggaac       420
   atctggtcaa accccccagc tcttccactc acagactctt accacagccc ccctgccggg       480
   aaccacgcct ctgtacccct ctccaatgac gccgatgact cccataactc ctgcgacacc       540
   ggcatccgag agctctggga tagtgccgca gctacagaat attgtgtcca cggtgaatct       600
   tggctgcaag cttgacctaa aaaccattgc gcttcgtgcc cgaaatgctg agtataatcc       660
   caagcgtttt gctgctgtga tcatgagaat aagagaacca cgtactactg cacttatatt      720
   cagctctgga aaaatggtgt gcacaggagc aaaaagtgaa gaacaatcca gactggcagc      780
   aaggaagtat gcgagagttg ttcagaaact gggttttcct gcaaaatttt tggattttaa      840
   aattcagaac atggtgggca gctgtgatgt gaaatttcct atcagattag aaggactggt      900
   actcacacac cagcagttca gcagctacga gccagaattg tttccaggct taatctacag      960
   aatgatcaag ccaagaattg ttctgcttat ttttgtttct ggaaaagtgg ttttaactgg     1020
   cgctaaagtt cgagcagaaa tctatgaagc atttgaaaac atctatccta ttttaaaggg     1080
   tttcagaaag acaacgtaac agtttctaca agtttcagag aaattggggg tacattttaa     1140
   aaggcattgc atatggcaca gcagtaacga gatggacttc agtgaaactg caacaccagg     1200
   acttggacta tttcccagtt agtgcctact tccccgatgc tcaaggggaa ttatattgat     1260
   tacgtctact gagttactgt gagttgcttt actgggctgt tcctggagca gtctcttcaa     1320
   gttttattta tattctagct tttaaatagt tttaatgcca gttctattaa cagaaaatcc     1380
   ataaattggt ttaagagaca actgcaactg tgtcactcag tgtataaacc acttaagttg     1440
   ccatgtatat atgttttaat tttcttccat aagaccatgg tttttataat tttaagaact     1500
   taagctttta aattttttca attttaattt tttttgtgcc agacacattc cctctttcca     1560
   gtattaagca agacagaata aatttattaa tgaaaatggc tcactgtaca tatatatata     1620
   atatatatat tcctcctctt tcctccttca gctccacatg tacaataaac cctgtttata     1680
   caattacggg aactgtctta ctgaatttta aaattatgca gctggtagag tgaaatgtat     1740
   taaacttttt ttttaatctg gatttataaa aagcacatac actactgtaa ctgacttgtc     1800
   agtttgagga gaatcttaac agtactctgg cttgcttacc ggtagccatg tccttggcac     1860
   catttagctt gaatatttct aggatgttaa aacagggggt gattggttaa agaattttgt     1920
   gttcaagtat gtgttgcttc acattcaaca gatgggattt atgcaagtct taaataacta     1980
   cacttgagtg aattggaaca aaagattctt gcttagcttc taggctttct ttaaattact     2040
   tttgtatttt accaactatg ccagtctcac tatattagtg ttcctgtaaa atgtcaaagc     2100
   ccttccaact agagtcagca tgtacataat tttccctttg aactgtgctt gaagcttaat     2160
   gggctgagaa ct                                                         2172
<210> 13
   <211> 1551
   <212> DNA
   <213> artificial sequence
<220>
   <223> cDNA du gène TBX5 du canard Pékin Anas platyrhynchos
<400> 13
   atggcggata gcgaggaagg ctttgggctc cccaccacgc cggctgactc ggaggccaag        60
   gagctgcagg ctgacgccaa gcaggaccag ctgggggccc ccagcaagcc ccccacctct       120
   ccccaggcag ccttcaccca gcagggcatg gaaggaatca aagtgttttt gcatgagcgg       180
   gagctgtggc tgaaatttca cgaagtgggg acggagatga tcataacaaa ggctggaagg       240
   cgcatgttcc ccagttacaa agtgaaggtc actggactta atccaaaaac taagtacata       300
   ctgttgatgg atattgtacc agcggatgac cacagataca aatttgcaga taataaatgg       360
   tccgtgacgg ggaaggcaga gccggccatg ccgcgcctct acgtgcaccc cgactccccc       420
   gccaccggag cgcactggat gcggcagctg gtgtccttcc agaagctgaa actcaccaac       480
   aaccacctcg accccttcgg acatatcatc ctcaactcca tgcacaagta ccagccccgg       540
   ctgcacatcg tgaaggcgga cgagaacaac ggcttcggct ccaagaacac cgccttctgc       600
   acccccgaga ccgccttcat cgccgtcacc tcctaccaaa accacaagat cacccagtta       660
   aagatcgaga acaacccctt tgccaaaggt ttccgcggca gcgacgacat ggagctccac       720
   aggatgtcca ggatgcagag taaggagtac ccggttgttc ccaggagcac ggtgagacag       780
   aaagtgtcct caaatcacag ccccttcagt ggtgagacca gggtcctttc cgcctcctcc       840
   aacctgggct cccagtacca gtgtgagaac ggggtgtcga gcacctccca ggacctgctg       900
   ccgcccgcca acccctaccc gatctcccag gagcacagcc agatctacca ctgcaccaag       960
   agaaaagatg aggaatgttc caccaccgag catccctaca agaagcccta catggagact      1020
   tctccggcag aagaggatcc cttctacagg tccagttacc cccagcagca gggactgaac      1080
   acttcataca ggactgaatc agcccagcgc caagcgtgca tgtacgccag ctccgctccc      1140
   cccacggacc ccgtgcccag cctggaagac atcagctgta acacgtggcc cagcgtgccc      1200
   tcctacagca gttgcacagt gtctgccatg cagccgatgg acaggttacc ctaccagcat      1260
   ttctctgccc acttcacctc ggggccgctg atgccccggc tcggcagcgt ggccaaccat      1320
   acctcccccc agataggaga cacccacagc atgttccagc accagacctc ggtttctcac      1380
   caacccatcg tccggcagtg tggaccccaa acgggcatcc agtccccccc cagcagcctc      1440
   cagcccgcag agttcctcta ttcccacggc gtgcctcgaa ccctctcgcc ccaccagtac      1500
   cactcggtgc acggcgtggg catggtgcca gagtggagcg agaacagcta a               1551
<210> 14
   <211> 1654
   <212> DNA
   <213> artificial sequence
<220>
   <223> cDNA du gène TYR du canard Pékin Anas platyrhynchos
<400> 14
   atgtctctgt ttgctatggg tttcctgcta gtcatccttc agccatccac cgggcagttc 60
   cccagggcct gtgcaaacac ccagagcttg ctgaggaagg agtgctgtcc tctctgggat 120
   ggagatgggt ccccttgtgg ggagagttcc aacagagggg cctgccaggc catccttctc 180
   tctcaggctc cactggggcc acaattccct ttctcgggag tggatgacag agaggactgg 240
   ccttctgtgt tttacaaccg gacctgcaag tgtaaaggta acttcatggg attcaactgt 300
   ggggagtgca agtttggttt ctcaggactc aactgcactg aaaggcgact gagaacgaga 360
   agaaacatct tccagctcac caacagtgag aaggacaagt tccttgccta ccttaacttg 420
   gcaaagaaca cccccagcca ggactatgtc attgccaccg gcacatatac tcagatgaac 480
   aacggctcca acctcatgtt cagaaacatc aacatgtatg atctctttgt gtggatgcat 540
   tattatgctt ctcgcgacac actcttaggt gggtccaatg tgtggcggga cattgatttt 600
   gcccacgaag cccctggttt tctgccttgg catcgtcttt ttctgctgct ctgggaacgt 660
   gagatccaga agataacagg tgatgagaac ttcaccatcc cctactggga ttggcgagat 720
   gcagaggact gtgtggtctg cacggatgaa tacatgggtg gcagacatcc aactaaccct 780
   aatttactca gcccagcatc atttttctcc tcctggcagg taatctgtac tcgatctgaa 840
   gagtacaaca gccaacagtc tttatgcaat gccactagtg aaggacctat actgcgaaat 900
   cctgggaaca atgacaaatc aaggactcca aggctcccat cttcagctga agttgaattt 960
   tgtctgtcac tgactcaata tgaatctgga tccatggata aaatggccaa ttacagcttc 1020
   cggaacactt tggaagaata tacaggcttt gctgatccac gcactgcaat atcaaacata 1080
   tctcaaagcg gtttgcataa tgcacttcac atctacatga atggctccat gtcccaagta 1140
   caaggctctg caaatgatcc tatcttctta ctacaccatg cttttgttga cagcattttt 1200
   gagcggtggt taagaagaca caggcccatc ctagaagttt acccagcagc caatgcaccc 1260
   atcggccaca atcgagaaaa ttacatggtc ccctttattc ctctttacag aaatggagaa 1320
   ttttttatac catcaagaga gctgggatat gactatgagt atctacaaga accagcactt 1380
   gattctttcc aggaattttt aataccatac cttgagcaag cccgtcagat ctggccctgg 1440
   ctggttggtg cagctgtgat tggaggtata gttactgctg tgctctctag cctcatcctg 1500
   gcctgcaaga agaaaagagc aggaacttct ccagagatac aacccttact cacagaaagc 1560
   gaagattaca acaatatatc ttatcaatcc catttctgaa gacctccagt aactcaatga 1620
   tgttagtaag gtgctaatca agcattcagt gttt 1654
<210> 15
   <211> 1629
   <212> DNA
   <213> artificial sequence
<220>
   <223> cDNA du gène TYRP1 du canard Pékin Anas platyrhynchos
<400> 15
   atcatgcagc tccccatgct gctgctcctt tccctgccac tgcttcttag catgctcaac      60
   aaagttggag ctcagttccc tcgccagtgt gctaccgttg agtctctgag gagtggcatg     120
   tgctgcccag actatttccc tgtatttggg cctggtactg accggtgtgg tgtgtctaca     180
   gggaggggac ggtgtgtgca ggtgactgta gactggcgac cccatggccc acagtacatc     240
   catgttcaac aaacctgcag gtgcagtggt aacttctctg gttataactg tgggtcatgt     300
   cgccctggat ggagtggacc taggtatgac aacagtaacc tcacagtcag gaggaatctt     360
   ttggatctga atgcagaaga gaggaggcgt tttgtgaatg ccttacacca agccaaggtg     420
   acaatccacc ctgacattgt tattgccaca aggagacgtg aagaaatatt tggaccagat     480
   ggaaacacac cacagtttga gaatatctcc atttataact actttgtgtg ggctcattat     540
   tattctgtca ggaagacttt tcttggtact gggcagcaga gttttggagg agttgatttc     600
   tctcatgagg gaccagcttt tgttacatgg cataggtacc atctgctgca gcttgaaaga     660
   gacatgcaga atatgttaca ggaccccact tttggccttc cctactggaa ctttgcaaca     720
   gggcaaaaca cctgtgatat ctgcttagat gacttgatgg gagctagaag caattttgat     780
   gtctctttga tcagtcagaa ttcgatcttt tctcagtggc gtgtgctctg tgaaagtata     840
   gaagactatg agaccttggg aaccatctgt aacagcactg aaggtggccc catccggagg     900
   aatcctgctg gaaatgttgc acggcctatg gtacaacgtc tcccagagcc tgaggatgtt     960
   gctcagtgtt tggaagttgg tgtatttgat actcctcctt tctactctaa ttcaacagac    1020
   agtttcttta agaatatata ttcagggtac agtgatcctt cagggaaata tgacccagca    1080
   gttcgaagtc ttcacaactt ggctcatcta tttttgaacg ggacaggagg acaaactcac    1140
   ttatcaccaa atgatccaat ttttgtcctc ctccacacat ttacagatgc tgtttttgat     1200
   gagtggctga gaaggtattc tgctggtaag atgtgctcat ggattttttg tggtcacagg     1260
   aaatcaactg cttcttatat ctcaacatat ccattggaga atgcccctat cggacacaac    1320
   cggcaataca acatggtgcc tttttggcct ccagtaacca ataatgagat gtttgttact    1380
   gcaccagaaa acctgggata cagctatgag gttgagtggc caggtcgggc tctccatgta    1440
   acagagatga taactattgc aatagtgact gcattggttc ttgttgcaat tatctttgct    1500
   gctgctgcat gtattgtacg tgtcaagaaa aataaggatg agttgcatca gcctcttctc    1560
   actgatcagt atcaacacta ttcagatgat tatgatggca tagcaacacc aagccagtct    1620
   gttgtatga                                                            1629
<210> 16
   <211> 21
   <212> DNA
   <213> artificial séquence
<220>
   <223> amorce sens du gène ALDH1 pour la PCR quantitative
<400> 16
   actgccagaa ccactcaaag a 21
<210> 17
   <211> 21
   <212> DNA
   <213> artificial sequence
<220>
   <223> amorce anti-sens du gène ALDH1 pour la PCR quantitative
<400> 17
   gtgatccaag ctcaaaagct g 21
<210> 18
   <211> 21
   <212> DNA
   <213> artificial sequence
<220>
   <223> amorce sens du gène CECR2, encore appelé gène CAT (figure 3) pour la PCR quantitative
<400> 18
   ggttctcaac tgttgctgga g 21
<210> 19
   <211> 21
   <212> DNA
   <213> artificial sequence
<220>
   <223> amorce antisens du gène CECR2 pour la PCR quantitative
<400> 19
   aaagactcag ggcgaagact c 21
<210> 20
   <211> 21
   <212> DNA
   <213> artificial sequence
<220>
   <223> amorce sens du gène EDN3 pour la PCR quantitative
<400> 20
   gctgaagctc agaagcaaga a 21
<210> 21
   <211> 21
   <212> DNA
   <213> artificial sequence
<220>
   <223> amorce antisens du gène EDN3 pour la PCR quantitative
<400> 21
   atattccctg ccaggttatg c 21
<210> 22
   <211> 21
   <212> DNA
   <213> artificial sequence
<220>
   <223> amorce sens du gène EDNRB pour la PCR quantitative
<400> 22
   ctgcacatca tcatcgacat c 21
<210> 23
   <211> 21
   <212> DNA
   <213> artificial sequence
<220>
   <223> amorce anti-sens du gène EDNRB pour la PCR quantitative
<400> 23
   actttggcac tccaattcct t 21
<210> 24
   <211> 21
   <212> DNA
   <213> artificial sequence
<220>
   <223> amorce sens du gène GRP143 pour la PCR quantitative
<400> 24
   gtccctgtcc agttgtgaaa a 21
<210> 25
   <211> 21
   <212> DNA
   <213> artificial sequence
<220>
   <223> amorce antisens du gène GPR143 pour la PCR quantitative
<400> 25
   tccgtgtaaa tcccttgtct g 21
<210> 26
   <211> 21
   <212> DNA
   <213> artificial sequence
<220>
   <223> amorce sens du gène LRP6 pour la PCR quantitative
<400> 26
   agaagcaaac tgccaagaca a 21
<210> 27
   <211> 21
   <212> DNA
   <213> artificial sequence
<220>
   <223> amorce antisens du gène LRP6 pour la PCR quantitative
<400> 27
   atggagccta tcgtgttgtt g 21
<210> 28
   <211> 21
   <212> DNA
   <213> artificial sequence
<220>
   <223> amorce sens du gène MLPH pour la PCR quantitative
<400> 28
   aggaagctgg atctctccaa g 21
<210> 29
   <211> 21
   <212> DNA
   <213> artificial sequence
<220>
   <223> amorce antisens du gène MLPH pour la PCR quantitative
<400> 29
   tgggactgat ttgtcaggaa c 21
<210> 30
   <211> 20
   <212> DNA
   <213> artificial sequence
<220>
   <223> amorce sens du gène OCA2 pour la PCR quantitative
<400> 30
   ccactgctga aggtctcctc 20
<210> 31
   <211> 20
   <212> DNA
   <213> artificial sequence
<220>
   <223> amorce antisens du gène OCA2 pour la PCR quantitative
<400> 31
   acttggcatg aaggttgtcc 20
<210> 32
   <211> 21
   <212> DNA
   <213> artificial sequence
<220>
   <223> amorce sens du gène PAX6 pour la PCR quantitative
<400> 32
   caccacttcc acaggtctca t 21
<210> 33
   <211> 21
   <212> DNA
   <213> artificial sequence
<220>
   <223> amorce antisens du gène PAX6 pour la PCR quantitative
<400> 33
   tgtgttacag catagggcac a 21
<210> 34
   <211> 21
   <212> DNA
   <213> artificial sequence
<220>
   <223> amorce sens du gène RPS17 pour la PCR quantitative
<400> 34
   aacgacttcc acaccaacaa g 21
<210> 35
   <211> 21
   <212> DNA
   <213> artificial sequence
<220>
   <223> amorce antisens du gène RPS17 pour la PCR quantitative
<400> 35
   ctcctcctcc tggagtttga t 21
<210> 36
   <211> 21
   <212> DNA
   <213> artificial sequence
<220>
   <223> amorce sens du gène SLC45A2 pour la PCR quantitative et semi-quantitative, cette amorce est également appelée Olig 1S
<400> 36
   ccagagggaa agagaaggct a 21
<210> 37
   <211> 21
   <212> DNA
   <213> artificial sequence
<220>
   <223> amoce antisens du gène SLC45A2 pour la PCR quantitative et semi-quantitative, cette amorce est également appelée Olig 1AS
<400> 37
   gatcactggc tgaacctacc a 21
<210> 38
   <211> 21
   <212> DNA
   <213> artificial sequence
<220>
   <223> amorce sens du gène TBP pour la PCR quantitative
<400> 38
   ccagctcttc cactcacaga c 21
<210> 39
   <211> 21
   <212> DNA
   <213> artificial sequence
<220>
   <223> amorce antisens du gène TBP pour la PCR quantitative
<400> 39
   ctcatgatca cagcagcaaa a 21
<210> 40
   <211> 21
   <212> DNA
   <213> artificial sequence
<220>
   <223> amorce sens du gène TBX5 pour la PCR quantitative
<400> 40
   aggatccctt ctacaggtcc a 21
<210> 41
   <211> 21
   <212> DNA
   <213> artificial sequence
<220>
   <223> amorce antisens du gène TBX5 pour la PCR quantitative
<400> 41
   aggtgaagtg ggcagagaaa t 21
<210> 42
   <211> 21
   <212> DNA
   <213> artificial sequence
<220>
   <223> amorce sens du gène TYR pour la PCR quantitative
<400> 42
   acacaggccc atcctagaag t 21
<210> 43
   <211> 21
   <212> DNA
   <213> artificial sequence
<220>
   <223> amorce antisens du gène TYR pour la PCR quantitative
<400> 43
   cctggaaaga atcaagtgct g 21
<210> 44
   <211> 21
   <212> DNA
   <213> artificial sequence
<220>
   <223> amorce sens du gène TYRP1 pour la PCR quantitative
<400> 44
   tggaaacaca ccacagtttg a 21
<210> 45
   <211> 21
   <212> DNA
   <213> artificial sequence
<220>
   <223> amorce antisens du gène TYRP1 pour la PCR quantitative
<400> 45
   gcaaagttcc agtagggaag g 21
<210> 46
   <211> 20
   <212> DNA
   <213> artificial sequence
<220>
   <223> amorce sens du gène SLC45A2 pour la PCR semi-quantitative, également appelée Olig 2S
<400> 46
   atgacccgcg ctccatgtca 20
<210> 47
   <211> 22
   <212> DNA
   <213> artificial sequence
<220>
   <223> amorce antisens du gène SLC45A2 pour la PCR semi-quantitative, cette amorce est également appelée Olig 2AS
<400> 47
   ctaccccaca tatcgaacac aa 22
<210> 48
   <211> 1632
   <212> DNA
   <213> artificial sequence
<220>
   <223> cDNA du gène SLC45A2 de la souche Barbarie Yeux Noire
<400> 48
   atgaccttaa ccaaacagag cttccacagg gcccttccac ccccttctga ggtggccatg 60
   accaacctgg accacaccag agggaaagag aaggctatgc agcaatccgt gaccaggact 120
   gggatggtgg tactgaagaa gcgaccgacg ggaaggctgg tcatgcacag catggcgatg 180
   tttggcaggg agttctgcta tgctgtggag gctgcctttg tcacgccggt gctgctcagt 240
   gtagggctgc ccaagagcct ctacagcctg gtgtggctca tcagcccgat cctgggcttc 300
   gtgctgcagc ccgtggtagg ttcagccagt gatcactgca cctgtagctg gggcaggagg 360
   cgaccgtaca ttctgggtct gggcatcata atgctggtag gcatggcttt gtacctcaat 420
   ggggacgaga tgatctcagc tttcatcaat gggagagaca agcagcagac atgggcgata 480
   gttgttacca tgctgggagt agtgcttttt gattttgcag ctgactttat tgatggtcct 540
   atcaaagcat atttatttga tgtctgctct catcaggata aagagaaggg tctgcattac 600
   catgccctct ttacaggctt aggaggagcc ctgggttacc tgacaggtgc tgtggattgg 660
   ggtcaaactg tactaggata ttccttaacg tcggaattcc aggtgatttt cctcttgtca 720
   gccttggttt tcctaatctg ccttactgta catctgcgca gtattcctga agtcccgctc 780
   agatataaca aagaagagac aaagttattg ttggaagtga ctgaccccag gaggtatagc 840
   tccatagagg aaatcaggaa tggatacatt gcatgtactg aaataaacgc tgcagcaaaa 900
   ccaaagaaac acgctgctat gtcatgtaca gagactcaaa ggcggatgac ccttaagtca 960
   ctcttgaaga tgcttttcag catgccatcc cactatcgct atctgtgcgt gagccacctc 1020
   tttggatgga tggctttcct gtccaacatg ctcttcttca cggatttcat gggacaggtt 1080
   gtataccgag gtagtcctta tgcacctcat aactccacag tttaccttac ctacagaaga 1140
   ggcgtagagg ttggatgctg ggggctgtgc atcaatgcaa tttcatcctc agtctattct 1200
   tacctgcaga aaatccttct gcaatacata ggattaaagg gactttattt cattggatac 1260
   ctactttttg gattaggtac tggattaatt ggcttgtttc ccaatgtcta ttccactctg 1320
   gctctgtgtt ccctctttgg tgtcatgtcc agcacactgt acacagtgcc attccaactc 1380
   attgcagagt atcacagaga agaagaggtc cagagtctgc gggaagacga ccaagacaca 1440
   gatcacaggc gagggaaggg cattgactgc gctgctctca cctgcatggt ccagctggcc 1500
   cagatcattc ttggtgtggg cttggggctc ttggttagtg tagcaggcag tgtggtcact 1560
   gtgatttctg catcgatggt gtcactgatc ggctgctgct ttgttgcttt ttgtgttcga 1620
   tatgtggggt ag 1632
<210> 49
   <211> 14231
   <212> DNA
   <213> artificial sequence
<220>
   <223> brin codant gène SLC45A2 du canard Pékin Anas platyrhynchos
<220>
   <221> misc_feature
   <222> (103)..(167)
   <223> n is a, c, g, or t
<220>
   <221> misc_feature
   <222> (335)..(352)
   <223> n is a, c, g, or t
<220>
   <221> misc_feature
   <222> (3116)..(3157)
   <223> n is a, c, g, or t
<400> 49
   cagaccaaat ccacccggca tatgacccgc gctccatgtc agccagagca ggcagcaccg      60
   acttgccagg ccggggcgag taaccagagc catgacttgt aannnnnnnn nnnnnnnnnn     120
   nnnnnnnnnn nnnnnnnnnn nnnnnnnnnn nnnnnnnnnn nnnnnnngag gtggccatga     180
   ccgacctgga ccacaccaga gggaaagaga aggctatgca gcaatccgtg accaggaccg     240
   ggatggtggt accgaagaag cgaccgacgg gaaggctggt catgcacagc atggcgatgt     300
   tcggcaggga gttctgctat gctgtggagg ctgcnnnnnn nnnnnnnnnn nngtgctgct     360
   cagtgtaggg ctgcccaaga gcctctacag cctggtgtgg ctcatcagcc cgatcctggg     420
   cttcgtgctg cagcccgtgg taggttcagc cagtgatcac tgcacctgta gctggggcag     480
   gaggcgaccg tacattctgg gtctgggcat cataatgctg gtaggcatgg ctttgtacct     540
   caatggggac gagatgatct caggtgagtg aggggaatgt gctgtgctga cagacaaaac     600
   tcatgccggt gttttgaatt ggaaagaaag gaggggttcc tttatttccc tgtaaaactt     660
   gggtgttaac attgggtagc acgttactcg gtggcactga agcagctgag gagcactgca     720
   atctatgttc agccctaatg ctgcatgtta tgtgccctgt atgtactgtt ctgtagtttg     780
   tgcatgtgat tacggtattt tgtatgttga tgtgcttgcc tggtcggttc atactctgct     840
   tgggcctaga aaagttttta agaactaaaa tctaccttat taagttaaat ggtttatgtt     900
   gtttgtatga tgtagtacaa atcttaagag aatgtctatt agtctgtatt ttagatgggt 960
   cggcattcaa atttatctgt aattcatctt ttaatgtagc tttaattatt gcagcacttg 1020
   cttggctcag gaatgtgtta tgctggtgaa taatacaaat cagaactcct tcgaaaccct 1080
   gttgcacctg gagtgtgcaa aatcagatag gagtgatgtc cccagatgct gttttatttt 1140
   agcttgatat ctaacttctc aaagatttgc tagaaagtca tcactatcat tgtggcttgt 1200
   gtctccttcc tgtggggagg agcgtgtttt cacagatttt ttctctgttt ccggatattg 1260
   ctgctgttgt atgtatgtgt aatcatttac ccagaaagag atgttagcca actcttgact 1320
   gtctataaca gagcaagggt ggaacaaaag gagtaaaaca aataaatact ccaaaatgca 1380
   agtattttag gctaccagaa catgcaatgg aagtgcatag agtagagaaa ggctagtgat 1440
   gctctcgatt gcagagcctt gagctgccaa gttcctgctt gtgtataaaa gtcaacctag 1500
   cacatctcag agctacgcaa actcaaaata tagttatcct gcttcttaga tgtcattctg 1560
   ttatgactga gctctcatgc gtaacagcgt tgtttagatt tggaggtttc tggaaagaaa 1620
   aatggggttt tgcaaaacat gacactattg ctgtcagaat gaggcttttt tttttttctt 1680
   tttgtagctt tcatcgatgg gagagacaag cagacgtggg cgatagttgt taccatgctg 1740
   ggagtagtgc tttttgattt tgcagctgac tttattgatg gccctatcaa agcatattta 1800
   tttgatgtct gctctcatca ggataaagag aagggtctgc attgccatgc cctcttcaca 1860
   ggtactcaga tcctttattt ttcttcctac tttgatggag gggaaggcag ttgcatggat 1920
   tttataatca ctcggtgcta atcttgaatg aactgatcat ctgattctgt gcttcagtca 1980
   cgggtctttt gttgctgggt tttcactact tcagagcaca gcaacttgtg attgctgcta 2040
   caagaattct gctgcagaag gctgcaggca taaacattta cagtagtata gccacggttc 2100
   aattaagggt aattaactat ttaaaaaaaa atgaagtaga gtttagtcat tttgttctga 2160
   ttttcttcaa gtttttggtg aaggaaaaat taagagaagg atcttgtttc cttggtgtag 2220
   cacttacaaa taagtatttt tgactcagca catgacagag caagtaacca gcagtacagc 2280
   tggtttctgt tcttcgacag tgctccaaca tatgctgccc tccactttag aaatagaagt 2340
   ttatgtaaaa tcactaactt ccagaaaata agaccaaggg gttcagtcca aaaacttgct 2400
   tcatgtgatt gcctttagac gtttgaaaag ttgctgagtt acggaaggaa agagttcatg 2460
   cagtgcagaa ggctttgccc tgtgttgagg aaacaaacag gcaatgcaca taaggctgcg 2520
   cacctgtagt gtgactttgg aggagcttcc agctgtgtct gagagcagtc actgtgaaag 2580
   gtctagacaa aaaacaggct agatgagaag tcatgcagtc tgtattgatg cagatttttg 2640
   tggcatctga accataccaa gagcagaagc acatggattc ggtggtggtt accccatttg 2700
   agagggaggt gtagtagcta ttggcaacag acagattttg gatatacgaa atagctgcat 2760
   cctggaacct gggcacagat tctaagagaa ccctgaagcc acatgggaat gaagtcagta 2820
   tgagccatta gaaacccaca gacaacattt taatcaatca ctcatttgga cagaatgagg 2880
   agtgagactc taagtctcct gccttcagca gaactcttta ctatttttcc tactaaaaag 2940
   ttttgatttc tacattcaga gctactgaac tgattcattt gtgtggtgaa aagtgatgta 3000
   agataaatca cattaaatgc aagcagaaca gatttctcca tattatttgg tccaacacct 3060
   tgtcactata tcaagatctt ctgtgcctaa acccatttgg atgggtagtc acccannnnn 3120
   nnnnnnnnnn nnnnnnnnnn nnnnnnnnnn nnnnnnntgg atgggtagtt gcccagctgg 3180
   agattctgca gccctcccaa ggcagcctgc tccagcattt cactggcctt gctgttaaat 3240
   gtttttccct aaaatctaat cttaatatac atttcctata gttaaaatat attactcctt 3300
   ttctctgtct ctattaaata cagaaagcat ccttccttcc cctttttaat tgcttctgtt 3360
   ttatgtatta ttatctttcc cctatttagt tccttctttt tacattaact atccccaaga 3420
   ctgctctcca atttgtcttt gccattttta aagttcaaac atgaaactgg atagcttgca 3480
   gatataaaac tgagacaagt agaagaaaga ctgcacatac ccaacacaca acacacagac 3540
   tgtgcattcc aatatcatga ttactttttg tcaaaacagt atgcaaaaaa gacctcgagc 3600
   atcaagcatt aactgctgtg caaacattta gtgtaagttt tcttccaaaa gatattaaca 3660
   gcttaattgt ttttcattag aaaagcaatg atacagtccc catccagtag ggcacaagga 3720
   aggagggctg tgatcagacc caagagggaa ataagtacag aaagcccaga atggaatgat 3780
   aatgacgtgg tgtttaggcg aagcatacgt ggttcttgtt acctctgtta ctttttgttg 3840
   gacagaatgg ttctgccgtg cattgtctcc agttctccta agcagaggag cagaaataca 3900
   tccaagtcct tcagatgctt ctgtgctggg tcacttccac atcctgcaga accacctggt 3960
   taacgaggtg gcaggactgt gcaaagctgc ctcccctgcc aaagctacgt gctggatgta 4020
   acagtgtaca cttgtacctt gtgcttctga aacagcttgc actctcatca gaaagaagga 4080
   attgtaaaac aatagactga attctttttt tattaacttt gagacacaag atagagtaaa 4140
   cagtaaaatg agaaggctaa attttctgag atggggaagc tcaataagag gaatttctca 4200
   tttatattcg ttcctttttg ttttcctttc ctagtggaga ggtacttgga ttttggtgat 4260
   tacaaaacaa cgggttttat ttcattcctt tcctaaaaaa acaacacaaa caaacaaaca 4320
   agcaaacaaa aaacaacatg gaagcagtat tactgttcag attctgctgt ataaagtaca 4380
   ttttacaaag tctgaagtac agatagtctt gatgtaatag gaaaattgtt ttttctatga 4440
   agtttcagaa aacacatgtt ccagtagagt tttattttgt gttactcact gtttcaaaga 4500
   aaagacagct tcaaagaaat gtttctgaat catacagaat attggctggc aaagagatgg 4560
   aggtccatgt tgctaaatat ttgggtcttt ttttgagagc tccatccacc agaaccaagg 4620
   atattttcca agagacattt tggtaactgc tcatatgtgt gaatagctca gtgttccact 4680
   tccttgtgat tctttccacg tataatgata tctgaagggt gtttattcaa tcattactag 4740
   agggaatcac atgttgaggg tattgtgtgc tgtttgttta ttttgtgaat accagtagat 4800
   tttttccccc tttttttttc ccccgtgtag gcttaggagg agccctgggt tacctgacag 4860
   gtgctgtgga ttggggtcaa accgtactag gatattcctt aacatcggaa ttccaggtga 4920
   ttttcctctt gtcagccttg gttttcctaa tctgccttac tgtacatctg cgcagtattc 4980
   ctgaagtccc gctcagatat aacaaagaag agacaaggtt attgttggaa gtgactgacc 5040
   ccaggaagta tagctccata gaggaaatca ggaatggata catagcatgt actgaaataa 5100
   atgctgcagc aaaaccaaag aagcacgctg ctacatcatg tacagaggta agtgcagaga 5160
   tgattttgta gcccatatca ttccatcctt ggagaccaac agccttttaa ttagctaatg 5220
   ctgactattt aaaatatacc cctttcacac acacgctcag taaaagaatc ctccaccagg 5280
   atgcagcatc agtagaaagt ctgctttctt ttcttttttt gttatttgta aagtatttcc 5340
   agcaagacag atggagacag actagtcctg atgctttgaa cagttggcag ggatgaggga 5400
   gaaccagatt caagtttcca cccttagtca gtttcttgta tcctcctcat ctgaaatttg 5460
   tcaagaggat ataatttgtc cttcagtcct ggcagaatta acaacacatt gtggacagga 5520
   gctacagacc attaatcttt gatataaatg tgcaactccc gttgatgtca acacgcttcc 5580
   acatttagta catatgaaga gttagttccc tggtctgata aacatttcag ttatgctact 5640
   gctcggtatt tcattgagga tttattgatg gtgttgattt gtttgaaatt gctacttaag 5700
   caacagtgcg acgtcacttc cagggtttta gttttattct gaagtgatgg tgtagctgtg 5760
   cctttgacag aattctaggt gggagagaat accctgtatt tgcaagtaat cactggtatt 5820
   ttgttcatgc tgaattctta tgagagatag cttcaagcta aggcagcaaa gaaaaggtga 5880
   acctacctga aaaatatctg gggaacacat ttatagaata gtcttaggaa cacgtagtgg 5940
   ttttcactgt tttgttgatg acttttaatg tggaaattct taccgccctt tcatggtatc 6000
   ctaggtatgc aaaggttaat atatatgttt ggttcctagt atggcagtaa gtaatgaaac 6060
   agttggctgt tttaatttca cttcttatct cttggcatta tgtgtatata tccctgacac 6120
   aattcttagg aaataagcta aaaattaatg agaacacaca gatgatttta agttagaaaa 6180
   cccgaattca tatttaaagt accaatataa tggatttatt atcttttact atttaacact 6240
   tgtattctag cagcacttgg taatcgcaac ccagataaga atattattat agcagacacc 6300
   acacaaagcc agaataaaag atgcacactg cctggtgagg atgccagaca cacggtgagg 6360
   gactggcagc aaagcagtag tgtcaggaga atatggctag cacactgcag tgttttcccc 6420
   gtttccctac ctcaggccaa cctgagcaga cagcatcctt cagcagcaca gttatctctg 6480
   agcatccttc aaattgtcag agacactctg tgtcattgac tgggcaatgc aagctccaaa 6540
   agaaaggaag ggcaatgaag aagagagggg gctgggctag cctcttgctt ggcaagaact 6600
   aataccagtg aatcagtagc atctgacacc cgaagaaaaa ttctgtcatg cactgcaggc 6660
   cagcttctgc tgttgccttt ctttctagga ttttagctgg acagcccatc agatgctcat 6720
   tgtgctttga tctgtctcat gtttgtttga tgtgcctggg cttgtgcaaa ttctggacac 6780
   tctcactacc gcagtggtct gcctcctgtg gcacatgttt tctgagttca ttcatcactg 6840
   taattacttc cttgaataaa tggtttgtct tcctgttttt ctgtgtctct tttttcaccg 6900
   caggcatcat ggccgtattt cttctaaatt aagggctatg ctcttacata ctaatagcgt 6960
   ccatggttat taatgagatt tgacctgtta tggtatgaac agaaatccaa aaatattctt 7020
   ctcttgttct ttattttgat gccaaattac tccggatcta tggccataaa gcttgtaata 7080
   tgaatcctta tgaatcagcc ttgagttgta gcaattttgt agaaaaaatg attaggtatt 7140
   gtgagatttt aaatgattgt gcattggagg gcactaaaca tatgtattaa acatttctgt 7200
   ttccgtttct actctttaaa atatctaaat agaaagaaag catgtctaac tgtcaaatta 7260
   ttatttggaa ttactgcccc tttaatttcc ctgaccgtat cctaatacct tgaaaatacc 7320
   ttagaaagcc tattttgaag aactttgtgg aagagttgaa caaattatat aagctaagtc 7380
   aaacaatagc aattatcctt gaaatgacgc taatgagtta gtgtggaact gccttgtttt 7440
   gttttttgta taattatagt tgaatagaat gattactctg tttaaaatac agttaaaaga 7500
   gacagtgata ctgagagaaa ttccaaggga tttagacaat atgcagttca aggtaaaagt 7560
   cagtgacttg aattttggca ttcctatgag gattaaaaac ctcacactgg agcaatgtgt 7620
   tttccaactt tatatgtggt gattgttcat agatcagctt gcaggataac ctagttcaaa 7680
   ggaaagcaga attgcgaaaa gtagctagag cactaaagga aggagaaaag ttatcagcta 7740
   acagcaaatt gaagacacaa ttcagctttc cagcaatgaa cataacatta aagttcatcc 7800
   acatttagtg gccatacagc tttcctcacc acctctccat tgttgccacc gaatattgct 7860
   agagaaaact ggaagtgttc catcatgcac agaatagtat taaaaaataa catttgtctt 7920
   tctctgaaag taaagaacca tgcaggagct tttaagtagt aaggactcta catgtatgtt 7980
   gcaaatgcct gacatcaagc acacctgaag caccgaagaa atcaagcgaa agaggtgatc 8040
   tgacctgatg tctgtgactg aaaacaaaac aaaataaata aataaaaaaa aaaaaaaaaa 8100
   aaaaaagagg ttttgggcac agggttcaga caaggaagag ggagataagg tagggcactg 8160
   aaggtggcaa gactatttgt ttttccatgt cagtgagatt tgttaaccca tacctataat 8220
   gtttttgatg ttgtattcag ttgttttgcc catgaggtgg gggtgttcag gtgctggagc 8280
   ttacattatc tggacaatag ggttttgggt ggtgctgcat ctcctgtcat gtatcacctc 8340
   ttcttgcctc ttgaggggga agatgtagtg gaaattccat agtgattata caccagaaga 8400
   ggttaagttt cttagggaac ataggacata gaaagaggaa tgggaacaaa gtctcagaac 8460
   tgcaatactc cgcaactgat atactttagt tctgaggaga tatttttggg tttcattatt 8520
   ttccatggca gctttaaatc tgtgatggaa agctaattgt ctcgaggaaa taattctttt 8580
   catgtttgtt ttcttttgtt ttttgagccc ctgctgaatg tgttttggca gaaaattttc 8640
   acttggttct agtggtaatc aataattcta ctgcctgtaa caacaagcag tgttgctaca 8700
   tctctgctgg tgtaccccag ctccccaagt ctctgtgatc tttcagatga ctttactata 8760
   caatggcttt acaagtcaaa tatacagtga aggacattca gtactcaagc aacccatcct 8820
   agcaataaca aataaaacac tttggaagtt tagcaatgaa aagatactga ggccttctcc 8880
   atttgccgcc agctatttta ttagctttca gggcaataat acagtcttat tgttaaatac 8940
   ttgcttttag actcaaaggc ggatgaccct taagtcactc ttgaagacgc ttttcagcat 9000
   gccatcccac tatcgttatc tgtgtgtgag ccacctgttt ggatggatgg ctttcctgtc 9060
   caacatgctc ttcttcacgg atttcatggg acaggtaaag gtgtaaaatc ctcttgcttt 9120
   tcttcccttg tagcatctcc tcctccacca aaaagaaaca agatattgtt acaaatctga 9180
   aggaatgtca gtgactccca tccagtatgc catgtttcct tgcctttttt tggtctcttt 9240
   aagtgagagg gagttttggt ttcctgcagt gtatatgaac acctaaaaga aaaattttgc 9300
   ttggctggat aggggactga ataagagaat gactgattgg aacgacgtat gtgcagcatg 9360
   aaagaaaaaa aaaaaagtaa ttatagataa tttagaaaat agatgctaaa gcaggaaagc 9420
   agaagcagag agagaaggag agtgggagat gggacactca aaggaaaaag ggcaaatgag 9480
   tggacagaac agaaaaaaat tgagggtaag aaatgaggac aaatctggta cgtgagagtc 9540
   tgggagaaat gagtgtgaaa tctgaagaac tgcaaatgga tgatggggac gctgcttgaa     9600
   agatttcttg ggtgctaatc atcaccccta ccagggatga ggccctgtct tgagtcccat     9660
   gttatggtaa gcaggaggta ttgtatacat gcaatgcatc cttgtctttt gtgacctcta 9720
   tggtctcttt caggttgtat accgaggtag tccttatgca cctcataact ccacagttta 9780
   ccttacctac agaagaggcg tagaggttgg atgctggggg ctgtgcatca atgcaatttc 9840
   atcctcagtc tattcttgta agtatccagg aatgtactcc aacactgttt taaatcagtg 9900
   tttatactgc tttcagtttg ccattgttta aaaaaataaa gtgatgccaa atattttttt 9960
   ttgcatgaaa actttaagaa atattacatt ctcagcagcg ttataaatct gtatcttgac 10020
   atgagcagtt cctgtttgga ccatgtgatg aggggtcact ttaagactaa ggcattgtgc 10080
   tgcatatgaa ctgaccctaa acactgaata ttatgctaaa ggtaaatgtc agagtaaaat 10140
   cgaagccgtg gttatcattc tgagatgctc tttcacctag cagacctgta gcattaggtt 10200
   ttcaaaggtc tgatggtctc tccccatctg catttctcac aaaatgacct tttcacagta 10260
   cagtagaagt tcttgccaaa aaaatgaacc ttgctctcaa aaattatgtg taaatactct 10320
   agcacagctt ctcaaataaa tctcaccata gctcttaccc aggatttaaa aatttcttgc 10380
   tatccaaaaa ctcaaattag gagcattttg ggaattgaag tgagcatcta aaagaagctt 10440
   tctcatttgg agcttaacta taggcattta tgtagcacct ctttcagctc agacaagggc 10500
   tgatgccaaa tgttcctgtt gtaatcaaac agcggaaatg tctgaggttg tcaatggggc 10560
   agttcttcca gaatggctgt taactacttc ttctcagagc ttggttatgt ctgaataaat 10620
   gtatctccag agacagttag cagaaagaag tattctgcag caagtttata accacagtaa 10680
   tttaaggcaa cttccaaaat caataccttt ctccttcatt tcatcttacg aaataaaact 10740
   ccttaaagga attccccaat gaaatgtgac ttattctgtt ctagtctcag cagcattttt 10800
   tctttctttc cccagacctg cagaaaatcc ttctgcaata cataggatta aagggacttt 10860
   atttcattgg atacctactt tttggattag gtactggatt aattggcttg tttcccaatg 10920
   tctattccac tctggctctg tgttccctct ttggtgtcat gtccagcaca ctgtacacag 10980
   tgccattcca actcattgca gagtatcaca gagaagaaga ggtaagttca atgtctaaag 11040
   aaggataaat ctgtttagtc tgttttggca gaacattgga tagaactggc aaacaggttt 11100
   taattgtaat ttcaaactat ttatggtgac atattatgaa cagaaagttt ctgacataaa 11160
   gatgttcaag actttattaa acaacactaa tattattagt tgtcatgtag ctacatgaca 11220
   cctcgcagct aatgtcccag ctccaggctc tccaaacaga gtcctgcatt gagacaaata 11280
   attatttatc atcccagctg aaacaaagcc agtcacaata catgttcagg caggaagagg 11340
   ataacagggc tccaatttgc attgctttca aagactgcct tcctgagaat caaatctaaa 11400
   ccactgctct gtgcaccatt gatctctcat ctgtgtgtgt atgcccttgt ttctaaatgc 11460
   aaggggctca ggctgagcag ctgccaaact caaattactt cagggaagca tagcctcttc 11520
   atattagctg aggagctgac ttctgaggcc ctaaagttaa taaccaggct gtaaaagctg 11580
   tcagaagttt cagttaactg caaagtctta caaaaaactg ttagctgata aagatatgca 11640
   attcaagtat gaacagctga acagctttat gtagttcagt aataaattgt actccacatt 11700
   cttgttctcc ctctaaatgt ctggggttgt tttttggtct tccacagcat gaaatcctgt 11760
   ttccaaagga aatctggaaa agcctgcatt ccttctatca tgttactgga cacagcaaga 11820
   tcttaaagtc ttgtaaagag tttagctctt taatggaaaa gttctctcaa attagtaaga 11880
   gagctggaaa caagaaggat taaggagaat tgggaggact taggaaaagt tctcattatg 11940
   tccagtgaca ttcgaagaaa ttaatataga aagtgttgaa cagttgaaaa gcaaaaaaaa 12000
   aatatatact tgagatcagc acaggactag attaagtcca tgagaacata tcctactgtt 12060
   ctatatgact tctaagcatc atcacctgaa tgatgagtac aagaaaaccc taaattaacc 12120
   ctaaattact ctgttgaatc ctaggaggat gtttcaaggt gaataaatat ttcatagatc 12180
   cgattcgcaa tttttcagtt aatttccctg tttcttccaa cagaaaacaa ttacaatgca 12240
   gaatttcaga gggatttaac aaaagacagg gtctgaaccc agtttggggt aggatcacaa 12300
   ccctaatctt aggccagcca gctcagagaa agggagtaaa gtagttataa gtaaattata 12360
   gcccatctct gactcagact tgtttcaaga tagaagtgaa atagaatacg ctgagtgtga 12420
   atggactttg tacttaatag gcttcaaatg tgagttgtga gagaactgat tgtttcttgt 12480
   ttggtgtatg gtacttgcta tgccagacgt taaatgataa ttctacgtga gatctgacat 12540
   taacagaatt aggtttgtcc caaaccttct ataataggaa aaaaaagata agtaaagagg 12600
   aaacatcaag tgcaagatcg tgttcattca cacattcata ttattgtggt cagaactact 12660
   gacacatagg aaccctgggc agagcacagc aaaacagttg taattccaaa gaaattgtgt 12720
   ctaaaattga aaatacttca gaggaaaggt ctgcattatt ccaaaacaaa gaacagactt 12780
   tttccctcag tatgcagtca accaaaatca tgtgctggtt tttaaaatgc tgccctttca 12840
   attcctgttc catgctgctg ttccctggga tccaaattca atttcatcta cgggattagg 12900
   aagacactgc ctccagtaat tttgagcttt gggtggtaac agctcaaaaa caatataaca 12960
   ggtcttgaaa aaaatatcct aatcacaatc aagagcattt ggatagagtc cagctcagct 13020
   agcacacagc acagaaaccc tctgtgtatt acaaagcaac tgtctccatt caactgcaag 13080
   aacaataaat acgtaaactt ttatgtaatt ttccaatgaa acagagcagt tggcacattg 13140
   cctgtgatca tgtccagttc agttttgaag gtctccgtgg gtggaagctc tacaacctct 13200
   ctggacagcc tgttccagtg ttcagccaac ctcacatggt gcttgtatat gagatgctca 13260
   tatagaataa aaccttttcc tcttctatgt gtgtcatttg aatgtcatag ctgataatgg 13320
   ccttctgtaa tgtagaagaa tcctagtctg tacattcatt ctcagacttt tttttttcag 13380
   aaataaaagc attacagtta gattttaata gtgaactcct ttatgtgctt tgaggcataa 13440
   cttcagagca caagagctcc attacaacaa attatgatct gtatgtgtgt gggacatact 13500
   atgtctcaat accccctgag gctgtcctta cagttggacc tgatgatctt gaaggtctct 13560
   tccattcctt ttccattctg tcattgctag atgattgtat gatgcagcca tgtgagagct 13620
   ggagtgaaaa catccttgac agggtgtcct gtacagtgaa tttttctcct tatattagtt 13680
   tgccagagct caagtagacc tgaacttcaa ggaaaggatt ggttttgctg atcattcttc 13740
   gattccttcc ttgaaagaag gggtagaggg aaaagttgga ggtgggagaa aagggacaat 13800
   ttggaaaact tactatcact tgtaaaatct gtttgaactt tgtaacctct gtgggacagg 13860
   caggagaaga tggaaaggaa aaggagagct attggcttgt tttgctgcac ttgaatgtca 13920
   tgacagtgca tcattagctg agaggtgcaa gttactagca gtgtttaagt tcccttccct 13980
   gaatcttgtt ttttgcttat ttgcaggtcc agagtctgca ggaagatgaa caagacacag 14040
   atcacaggcg agggaagggc attgactgcg ccgctcttac ctgcatggtc cagctggccc 14100
   agatcattct tggtgtgggc ctggggctcc tggttagtgt agctggcagt gtggtcactg 14160
   tgatttctgc atcgatggtg tcactgatcg gctgctgctt tgttgctttt tgtgttcgat 14220
   atgtggggta g 14231
<210> 50
   <211> 29
   <212> DNA
   <213> artificial sequence
<220>
   <223> amorce sens du gène SLC45A2 pour la PCR génomique, dit Olig 1000 S
<400> 50
   ttcagcagat aatcactctg acgaattgc 29
<210> 51
   <211> 27
   <212> DNA
   <213> artificial sequence
<220>
   <223> amorce sens du gène SLC45A2 pour la PCR génomique, dite Olig 800 S
<400> 51
   acttacttta cagcacaccc aggtttt 27
<210> 52
   <211> 29
   <212> DNA
   <213> artificial sequence
<220>
   <223> amorce sens du gène SLC45A2 pour la PCR génomique, dite Olig 500 S
<400> 52
   aaataatcag tttatcgagg gtggcacag 29
<210> 53
   <211> 21
   <212> DNA
   <213> artificial séquence
<220>
   <223> amorce sens du gène SLC45A2 pour la PCR génomique, dite Olig ADN 1S
<400> 53
   tgtgagtccc tcccccactg c 21
<210> 54
   <211> 25
   <212> DNA
   <213> artificial sequence
<220>
   <223> amorce antisens du gène SLC45A2 pour la PCR génomique, dite Olig ADN 2AS
<400> 54
   ctcgtcccca ttgaggtaca aagcc 25
<210> 55
   <211> 22
   <212> DNA
   <213> artificial sequence
<220>
   <223> amorce sens du gène SLC45A2 pour la PCR génomique, dite Olig ADN 4S
<400> 55
   aggagccctg ggttacctga ca 22
<210> 56
   <211> 26
   <212> DNA
   <213> artificial sequence
<220>
   <223> amorce antisens du gène SLC45A2 pour la PCR génomique, dite Olig ADN 4AS
<400> 56
   atacttcctg gggtcagtca cttcca 26
<210> 57
   <211> 22
   <212> DNA
   <213> artificial sequence
<220>
   <223> amorce sens du gène SLC45A2 pour la PCR génomique, dite Olig ADN 5S
<400> 57
   agactcaaag gcggatgacc ct 22
<210> 58
   <211> 25
   <212> DNA
   <213> artificial sequence
<220>
   <223> amorce antisens du gène SLC45A2 pour la PCR génomique, dite Olig ADN 5AS
<400> 58
   cctgtcccat gaaatccgtg aagaa 25
<210> 59
   <211> 28
   <212> DNA
   <213> artificial sequence
<220>
   <223> amorce antisens du gène SLC45A2 pour la PCR génomique, dite Olig ADN 8AS
<400> 59
   ctaccccaca tatcgaacac aaaaagca 28
<210> 60
   <211> 23
   <212> DNA
   <213> artificial sequence
<220>
   <223> amorce sens AWS03 S
<400> 60
   acagtttgtc tgtctccggg gaa 23
<210> 61
   <211> 23
   <212> DNA
   <213> artificial séquence
<220>
   <223> amorce anti-sens ASP3-CJ AS
<400> 61
   agctggactt cagtgcatct tct 23
<210> 62
   <211> 18
   <212> DNA
   <213> artificial sequence
<220>
   <223> amorce sens INT-F
<400> 62
   atagaaacaa tgtgggac 18
<210> 63
   <211> 18
   <212> DNA
   <213> artificial sequence
<220>
   <223> amorce antisens INT-R
<400> 63
   ctctgtctgg aaggactt 18

## Description

La présente invention a pour objet un procédé d'identification d'une population de palmipèdes, en particulier d'une population spécifique de canards Barbarie présentant des Yeux Rouges (BYR) à l'aide de la détection moléculaire de différentes formes du gène *SLC45A2.*

Les palmipèdes appartiennent à l'ordre des Ansériformes et à la famille des Anatidés qui rassemble un grand nombre d'espèces, telles que les cygnes, les oies ou encore les canards. Ces deux dernières espèces, à savoir les oies et les canards, présentent un intérêt agronomique et économique. En effet, les canards et les oies sont domestiqués et élevés pour leur chair, la production de foie gras et la production de leurs oeufs.

De nombreuses souches, maintenues en lignées, existent en élevage. Ces lignées sont obtenues au cours de processus de sélection et de croisement de différentes espèces domestiques. Par lignée, on entend une population animale, fermée depuis au moins deux reproductions généalogiques, sur laquelle certains critères sont mesurés et sélectionnés. Sélectionner consiste à choisir au sein d'une population fermée, les reproducteurs les plus aptes à faire évoluer ou à maintenir les caractères quantitatifs et qualitatifs de leur descendance, caractères évalués par des critères de sélection. Ces critères sont un ensemble de mesures et d'éléments objectifs recueillis sur les individus candidats ou sur leurs apparentés qui permettent de quantifier ou de définir les aptitudes d'un candidat. Au cours de cette sélection généalogique, différentes souches sont isolées et se caractérisent par différents critères, comme leur vitesse de croissance, leur facilité de consommation des aliments, leur indice de consommation, leur capacité de reproduction, leur potentiel de fertilité, leur mortalité embryonnaire, etc. Ainsi, des lignées nouvelles présentant des caractéristiques phénotypiques et génotypiques particulières sont obtenues en élevage.

Les palmipèdes posent un problème technique d'identification du sexe à l'éclosion. Aucun signe morphologique ou phénotypique ne distingue, par exemple, un caneton mâle d'un caneton femelle. Or la valorisation économique des mâles et des femelles est différente dans les différentes activités mentionnées. Les mâles sont en effet privilégiés pour la production de la chair et du foie gras et les femelles pour celle des oeufs.

Une solution consiste à déterminer manuellement juste après l'éclosion le sexe par retournement du cloaque afin d'identifier visuellement les organes génitaux des palmipèdes. Cette approche, maîtrisée par beaucoup de producteurs est actuellement mise en oeuvre et nécessite un savoir faire très particulier et une main d'oeuvre importante.

Une autre solution est l'identification des palmipèdes par sexage automatique comme mentionné dans la demande FR 2 921 542 qui décrit un procédé et une installation pour le sexage des volatiles vivants de la famille des Anatidés, en particuliers des canetons.

Il existe, chez les palmipèdes, des souches colorées et des souches blanches notamment dans l'espèce de canard Pékin *Anas platyrhynchos* et dans l'espèce Barbarie *Cairina moschata,* les deux espèces domestiques de canard les plus fréquemment rencontrées. De nombreuses souches de canard commun, appartenant à l'espèce *Anas platyrhynchos* sont également identifiées parmi lesquelles le canard Colvert sauvage, le canard de Rouen, le canard de Challans. Les oies, telles que les oies cendrées (*Anser anser*), les oies cygnoïdes (*Anser cygnoides*) sont également des espèces sauvages et domestiques et il existe différentes souches colorées ou blanches d'oie.

Les palmipèdes présentent des yeux dont la coloration apparait noire dès l'éclosion et ce phénotype Yeux Noirs (YN) est considéré comme le caractère de phénotype sauvage. Jusqu'à présent, il n'existe pas pour les palmipèdes de publication, ni dans la littérature scientifique, ni dans la documentation de souches commerciales, d'autres phénotypes que cette couleur noire des yeux.

La mise en place de la pigmentation de l'oeil en général s'effectue au cours du développement embryonnaire sous l'action conjuguée de production de pigments, de leur transport et accumulation dans des types cellulaires bien particuliers, les cellules de la zone pigmentée. Chez les mammifères et les oiseaux, la pigmentation des yeux, des phanères et de la peau résulte de la synthèse de deux types de polymères, la mélanine/l'eumélanine de coloration noire-marron et la phéomélanine de coloration jaune-rouge. La tyrosinase est l'enzyme responsable de la première étape de la synthèse de ce pigment dans les mélanosomes ont été ainsi identifiés dans la littérature comme étant impliqués dans ces processus et sont donc directement susceptibles d'être responsables du caractère albinos avec des phénotypes plus ou moins sévères selon les espèces animales ou les individus, mais aucun ne traite le cas des Anatidés comme les canards Pékin, Barbarie ou les oies.

Dans une lignée de l'espèce canard Barbarie sélectionnée par la Demanderesse sur des critères de chairs (poids vif, rendement viande, etc) depuis quinze générations en population fermée, il a été identifié lors d'une éclosion pédigrée, quelques canetons avec une couleur de duvet un peu plus clair et des yeux rouges (YR). Il s'est avéré que ces canetons à yeux rouges (YR) étaient uniquement des femelles. Le phénotype est qualifié d'Albinos, le terme se référant à un défaut de pigmentation. En effectuant plusieurs croisements en retour, des mâles homozygotes de phénotype Albinos ont été obtenus pour ensuite créer la lignée pure Barbarie Yeux Rouges (BYR) de phénotype albinos stable et pérenne pour les mâles et les femelles. Jusqu'à présent, aucune publication n'a fait état d'autres canards présents dans les mélanocytes. Dans ces différentes espèces animales et chez l'homme également, l'apparition d'un défaut de pigmentation des yeux est souvent associée au caractère albinos. Il existe deux formes majeures d'albinisme, l'albinisme oculaire-cutané (OCA) et l'albinisme oculaire (OA) en général moins sévère. Des troubles de la vision plus ou moins importants sont souvent associés à ce phénotype. Des modèles de poulet sont même étudiés pour suivre la dégradation de la rétine souvent observée dans les cas d'albinisme.

Dans la littérature, des altérations de séquences de gène ont été répertoriées comme associées au phénotype albinos dans différentes espèces dont le poulet. On peut citer par exemple les gènes *CECR2, EDN3* et *EDNRB* (Nataf et al., 1998), *GPR143* (Lopez et al., 2008), LRP6 (Hunter et al.,2004), *MITF* (Mochii et al., 1998), *MLPH* (Vaez et al., 2008), *OCA2* (Sturm, 2009), *PAX6* (Bhat et al., 2004), *SLC45A2* (Gunnarsson et al., 2007), *TBX5* (Golz et al., 2008), *TYR* (Chang et al., 2006) et *TYRP1* (April et al., 1998).

Le document GRAF JUSTIN ET AL: "Promoter polymorphisms in the MATP (SLC45A2) gene are associated with normal human skin color variation", HUMAN MUTATION, vol. 28, no. 7, juillet 2007 (2007-07), pages 710-717, décrit des amorces pour l'amplification d'une séquence dans la région du promoteur du gène SLC45A2 chez l'homme.

Le document TSUBOI KIE ET AL: "Oculocutaneous Albinism in Suncus murinus : Establishment of a Strain and Identification of Its Responsible Gene", EXPERIMENTAL ANIMALS (TOKYO), vol. 58, no. 1, janvier 2009 (2009-01), pages 31-40, décrit une altération du gène SLC45A2 comme associé au phénotype albinos chez la musaraigne musquée.

En conséquence, les défauts de pigmentation résultent à la fois chez les mammifères et les oiseaux d'altérations génétiques dans la voie de synthèse, de transport ou d'accumulation de la mélanine. Différents gènes Barbarie à Yeux Rouges (BYR) pouvant exister, autre que ceux sélectionnés par la Demanderesse.

Dans le processus de sélection de nouvelles lignées, il peut être avantageux de réaliser des croisements entre des individus à yeux noirs (BYN) d'une souche donnée avec des individus de la lignée pure Barbarie Yeux Rouges (BYR), en particulier en utilisant les mâles BYR dont seules les femelles issues de ce croisement auront les yeux rouges et pourront ainsi être immédiatement reconnues phénotypiquement. Ce caractère autosexable pourra donc être utilisé au cours du processus de sélection.

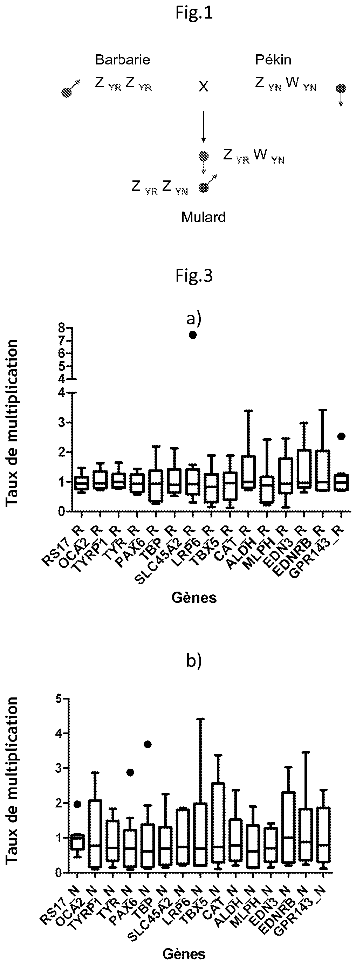

Par exemple, dans le processus de production de foie gras et de viande, le mulard est le résultat du croisement entre un mâle Barbarie et une femelle Pékin. Or en utilisant les mâles Barbarie YR croisés avec des femelles Pékin YN, il apparaît que seules les femelles mulardes résultantes présentent le phénotype YR. Les mâles présentent le phénotype YN. Ce caractère récessif lié au sexe apparaît ainsi dans le schéma du croisement illustré sur la figure 1. Son utilisation à des fins de sélection et à des fins commerciales est ainsi particulièrement importante pour cribler les animaux issus du croisement et ainsi sélectionner les mulards mâles YN qui seront utilisés pour faire du foie gras.

L'invention a donc pour but de fournir un procédé d'identification permettant de certifier l'appartenance ou la non-appartenance à une population donnée d'un échantillon prélevé sur un palmipède, cette population donnée présentant le phénotype YR comme le canard Barbarie YR ou le mulard femelle YR. Ce procédé d'identification pouvant être réalisé en l'absence de la visualisation du phénotype YR.

Un des aspects de la présente invention est de proposer un procédé d'identification d'un phénotype des canards et des canetons par une approche moléculaire à l'aide de la détection de différentes formes du gène *SLC45A2.*

Un autre aspect de l'invention est d'identifier le sexe des canards et des canetons sur la base de cette identification des formes du gène *SLC45A2.*

A cet effet, la présente invention concerne un procédé d'identification de populations de palmipèdes, ledit procédé étant effectué à partir d'un échantillon biologique prélevé sur l'animal, tel qu'une plume, un échantillon de sang ou des fèces, et permettant de vérifier, l'appartenance ou la non-appartenance de l'animal sur lequel a été prélevé ledit échantillon biologique à une population de palmipèdes, ce procédé comprend :
- l'amplification de la région promotrice du gène *SLC45A2* dudit échantillon dans un milieu réactionnel comportant au moins un couple d'amorces par une PCR génomique, de sorte à obtenir ou non des produits d'amplifications,
- la séparation des produits d'amplification par électrophorèse sur gel selon leur taille, et
- la comparaison des profils d'électrophorèse obtenus à partir de différentes populations de palmipèdes, telles que des canards Barbarie à Yeux Noirs (BYN), des canards Barbarie à Yeux Rouges (BYR), des canards Pékin ou encore des oies, aux fins d'identification de bandes dont le polymorphisme est génétiquement lié au locus du gène *SLC45A2* qui participe à la coloration des yeux et permettant de retrouver, le cas échéant, l'origine de parenté dudit échantillon.

Par l'origine de parenté, on entend qu'il est possible, le cas échéant, de retrouver la lignée parentale dont est issu l'échantillon. Par exemple, s'il s'agit d'un mulard mâle ou femelle, il est possible de savoir si le père était de l'espèce canard Barbarie à yeux rouge (BYR).

Par « population » de palmipède, on entend un groupe de palmipèdes d'une même espèce, à savoir canards Pékin, Barbarie ou oies *Anser anser* ou oies *Anser cygnoides* et présentant éventuellement en plus des caractéristiques phénotypiques particulières comme par exemple la couleur des yeux : YR ou YN (une population peut correspondre à des canards Barbarie YR ou des canards Barbarie YN ou des canards Pékin).

Pour la présente invention, une amorce est une courte séquence d'oligonucléotides qui, hybridée avec une matrice d'acide nucléique, permet à une polymérase de synthétiser un nouveau brin de l'ADN. Le brin produit à partir de l'amorce est complémentaire au brin utilisé comme matrice..En particulier une amorce sens est utilisée avec une amorce anti-sens afin d'amplifier un fragment d'acide nucléique (amplicon) par PCR.

La présente invention découle de la mise en évidence par la Demanderesse de l'altération de la région promotrice d'un gène majeur de la coloration des yeux chez les palmipèdes, à savoir le gène *SLC45A2,* et du fait que le polymorphisme de ce gène chez les palmipèdes et notamment chez les canards Barbarie est une caractéristique de l'appartenance à la lignée BYR ou BYN.

Selon une première variante de réalisation, le au moins un couple d'amorces 5'-3' utilisé est défini de la manière suivante:
- l'amorce 5' est choisie parmi l'amorce Olig-1000 représentée par la séquence SEQ ID NO : 50 suivante:
   5' TTCAGCAGATAATCACTCTGACGAATTGC 3' ou toute séquence complémentaire ou dérivée de cette séquence SEQ ID NO : 50, notamment par suppression et/ou substitution et/ou addition d'un ou de plusieurs nucléotides,
- l'amorce 3' est choisie parmi l'amorce Olig ADN 2AS représentée par la séquence SEQ ID NO : 54 suivante:
   5' CTCGTCCCCATTGAGGTACAAAGCC 3' ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1990 bp sur ledit profil électrophorétique.

Selon une deuxième variante de réalisation, le au moins un couple d'amorces peut être également défini de la manière suivante:
- l'amorce 5' est choisie parmi l'amorce Olig-800 représentée par la séquence SEQ ID NO : 51 suivante:
   5' ACTTACTTTACAGCACACCCAGGTTTT 3' ou toute séquence complémentaire ou dérivée de cette séquence SEQ ID NO : 51, notamment par suppression et/ou substitution et/ou addition d'un ou de plusieurs nucléotides,
- l'amorce 3' est choisie parmi l'amorce Olig ADN 2AS représentée par la séquence SEQ ID NO : 54 susmentionnée ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1790 bp sur ledit profil électrophorétique.

De préférence, le milieu réactionnel comprend un couple d'amorces apte à permettre l'identification du sexe dudit palmipède.

Selon une caractéristique de l'invention, ledit couple d'amorces apte à permettre l'identification du sexe est défini de la manière suivante:
- l'amorce 5' est choisie parmi l'amorce AWS03 S représentée par la séquence SEQ ID NO : 60 suivante :
   5' ACAGTTTGTCTGTCTCCGGGGAA 3' ou toute séquence complémentaire ou dérivée de cette séquence SEQ ID NO : 60, notamment par suppression et/ou substitution et/ou addition d'un ou de plusieurs nucléotides,
- l'amorce 3' est choisie parmi l'amorce USP3 -CJ AS représentée par la séquence SEQ ID NO : 61 suivante :
   5' AGCTGGACTTCAGTGCATCTTCT 3'
   ou toute séquence complémentaire ou dérivée de cette séquence SEQ ID NO : 61, notamment par suppression et/ou substitution et/ou addition d'un ou plusieurs nucléotides.

La présente invention a également pour objet un kit d'identification d'individus ou de populations de palmipèdes par identification de la région promotrice du gène *SLC45A2* mis en oeuvre par le procédé d'identification susmentionné, kit comprenant les réactifs appropriés pour l'amplification de la région promotrice du gène *SLC45A2* à partir d'un échantillon prélevé dans l'environnement (fèces, plume, etc.) ou sur un animal issu d'une population choisie parmi les palmipèdes, caractérisé en ce que le kit comprend :
- un couple d'amorce permettant d'amplifier un fragment de la région promotrice du gène *SLC45A2,*
- des dNTPs et des polymérases présentes en large excès dans le milieu réactionnel
- les composants (tampon, eau, sels, ...) nécessaires à la réalisation de la réaction d'amplification par PCR

Selon une première variante de réalisation, le au moins un couple d'amorces 5'-3' utilisé est défini de la manière suivante:
- l'amorce 5' est choisie parmi l'amorce Olig-1000 représentée par la séquence SEQ ID NO : 50 suivante :
   5' TTCAGCAGATAATCACTCTGACGAATTGC 3' ou toute séquence complémentaire ou dérivée de cette séquence SEQ ID NO : 50, notamment par suppression et/ou substitution et/ou addition d'un ou de plusieurs nucléotides,

- l'amorce 3' est choisie parmi l'amorce Olig ADN 2AS représentée par la séquence SEQ ID NO : 54 suivante :
   5' CTCGTCCCCATTGAGGTACAAAGCC 3' ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1990 bp sur ledit profil électrophorétique.

Selon une seconde variante de réalisation, le kit comprend en outre un autre couple d'amorces défini de la manière suivante:
- l'amorce 5' est choisie parmi l'amorce Olig-800 représentée par la séquence SEQ ID NO : 51 suivante :
   5' ACTTACTTTACAGCACACCCAGGTTTT 3' ou toute séquence complémentaire ou dérivée de cette séquence SEQ ID NO : 51, notamment par suppression et/ou substitution et/ou addition d'un ou de plusieurs nucléotides,
- l'amorce 3' est choisie parmi l'amorce Olig ADN 2AS représentée par la séquence SEQ ID NO : 54 susmentionnée ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1790 bp sur ledit profil électrophorétique.

De préférence, le kit comprend un couple d'amorces apte à permettre l'identification du sexe dudit palmipède.

Préférentiellement, ledit couple d'amorces apte à permettre l'identification du sexe est définie de la manière suivante :
- l'amorce 5' est choisie parmi l'amorce AWS03 S représentée par la séquence SEQ ID NO : 60 suivante :
   5' ACAGTTTGTCTGTCTCCGGGGAA 3' ou toute séquence complémentaire ou dérivée de cette séquence SEQ ID NO :60, notamment par suppression et/ou substitution et/ou addition d'un ou de plusieurs nucléotides,
- l'amorce 3' est choisie parmi l'amorce USP3 -CJ AS représentée par la séquence SEQ ID NO : 61 suivante :
   5' AGCTGGACTTCAGTGCATCTTCT 3' ou toute séquence complémentaire ou dérivée de cette séquence SEQ ID NO :61, notamment par suppression et/ou substitution et/ou addition d'un ou de plusieurs nucléotides.

D'autres caractéristiques et avantages de l'invention sont donnés dans les exemples qui suivent, dans lesquels il est fait référence aux figures 1 à 10 qui concernent respectivement,
- La figure 1 représente le schéma de croisement entre un canard Barbarie mâle et une cane Pékin femelle ;

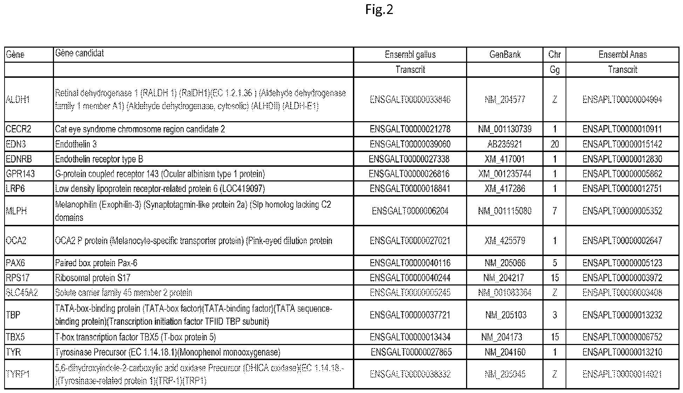

- La figure 2 est un tableau représentant l'ensemble des gènes étudiés impliqués dans certaines formes d'albinisme dans différentes espèces ou impliqués dans la pigmentation des yeux et des phanères;
- La figure 3 représente deux schémas montrant les profils d'expression des gènes de la figure 2 étudiés par PCR quantitative, le gène CECR2 du tableau de la figure 2 étant nommé gène CAT sur cette figure. En particulier, ces schémas indiquent la répartition par rapport à la moyenne de l'expression des gènes dans une cohorte de 11 échantillons prélevés sur des individus indépendants appartenant à la lignée Barbarie BYR (Figure 3a) et de 11 échantillons prélevés sur des individus indépendants appartenant à la lignée Barbarie BYN (Figure 3b) ;

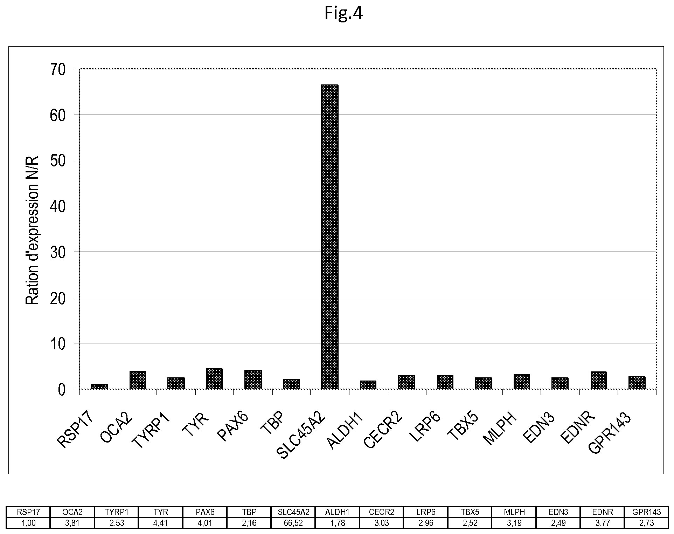

- La figure 4 est un graphique illustrant le ratio YN/YR de l'expression relative des différents gènes mentionnés entre les souches BYN et BYR, obtenus à partir de l'analyse de 23 échantillons prélevés sur des individus indépendants;

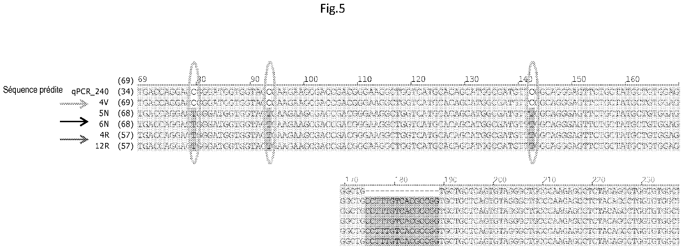

- La figure 5 représente la séquence des amplicons obtenus à partir des Canards Pékin (4V), de deux individus (5N et 6N) de canards de la souche Barbarie BYN et de deux individus (4R et 12R) de canards de la souche Barbarie BYR;

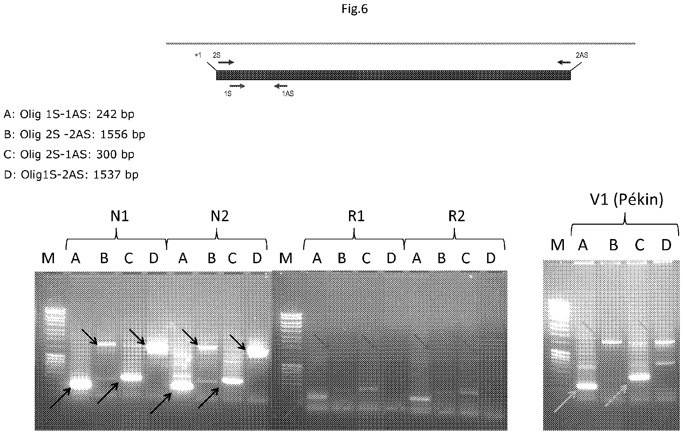

- La figure 6 représente le profil électrophorétique résultant de l'amplification par PCR semi-quantitative du niveau relatif d'expression du gène *SLC45A2* dans les mRNAs obtenus à partir des yeux de deux embryons de canards de la souche Barbarie BYN (N1 et N2) et de deux embryons de canards de la souche Barbarie BYR (R1 et R2) et d'un canard Pékin (V1) en utilisant 4 couples d'amorces: Olig1S - 1AS, Olig2S - 2AS, Olig2S - 1AS et Olig1S - 2AS ;

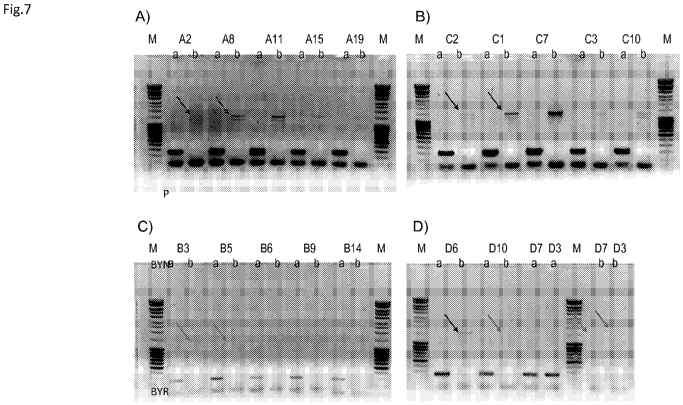

- La figure 7 représente des profils électrophorétiques résultant de l'amplification par PCR semi-quantitative du niveau relatif d'expression du gène *SLC45A2* dans les mRNAs obtenus à partir des yeux de cinq embryons différents issus des souches Barbarie BYN (A), Barbarie BYR (C), de Mulards issus de père Barbarie BYN de phénotype YN (B) et de Mulards issus de père Barbarie BYR de phénotype YR (D) en utilisant l'amorce Olig 2S et Olig 2AS (bande sur le profil électrophorétique à 1556 bp);

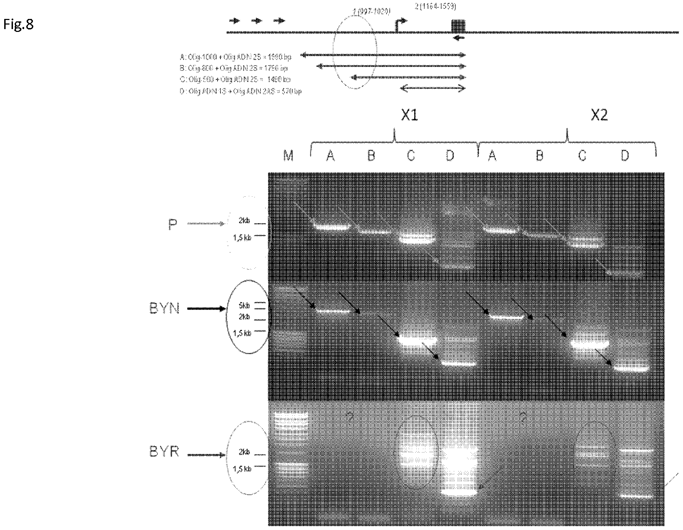

- La figure 8 représente le profil électrophorétique résultant de l'amplification par PCR génomique du promoteur du gène *SLC45A2* à partir de l'ADN génomique de deux individus de la souche Barbarie BYN, de deux individus de la souche Barbarie BYR et d'un canard Pékin YN (P) en utilisant 4 couples d'amorces : Olig-1000 et Olig ADN 2AS ; Olig-800 et Olig ADN 2AS ; Olig-500 et Olig ADN 2AS, et Olig ADN 1S et Olig ADN 2AS ;

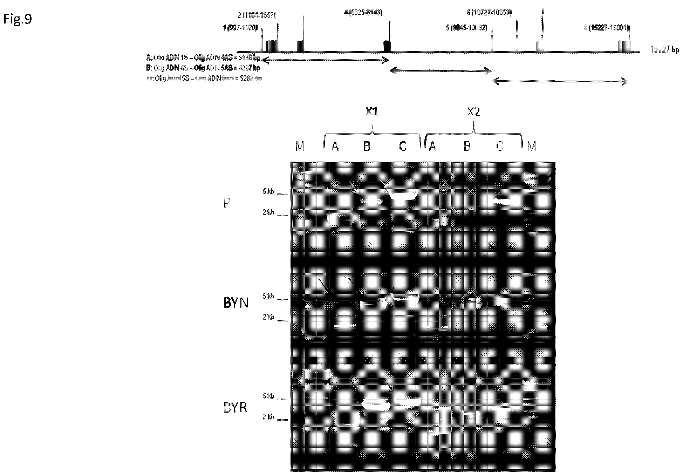

- La figure 9 représente le profil électrophorétique résultant de l'amplification par PCR génomique de différents fragments du gène *SLC45A2* à partir de l'ADN génomique de deux individus de la souche Barbarie BYN, de deux individus de la souche Barbarie BYR et d'un canard Pékin (YN) en utilisant 3 couples d'amorces : Olig ADN 1S et Olig ADN 4AS, Olig ADN 4S et Olig ADN 5AS, et Olig ADN 5S et Olig ADN 8AS ;

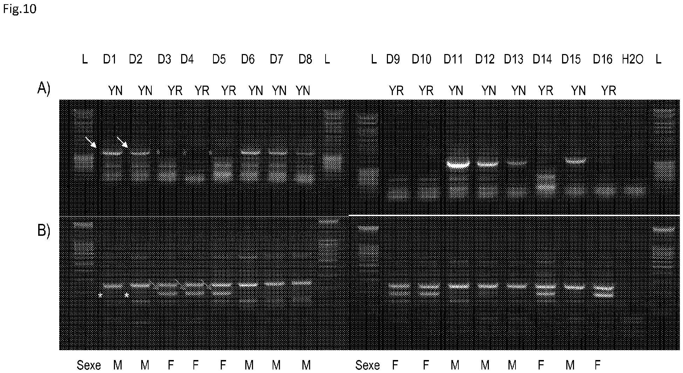

- La figure 10 représente des profils électrophorétiques résultant de l'amplification par PCR génomique du promoteur du gène *SLC45A2* en utilisant le couple d'amorce Olig-800 et Olig ADN 2AS à partir de l'ADN génomique de 16 échantillons (D1 à D16) issus d'individus Mulards issus du croisement d'un mâle de la souche Barbarie BYR de phénotype YR et d'une femelle Pékin aux yeux noirs pris au hasard en suivant le procédé selon l'invention. L'amplification est observée ou est absente (figure 10 A) en parfaite corrélation avec le sexe des individus (figure 10 B).

### I) Identification de l'origine génétique du phénotype Yeux Rouges (YR) chez un palmipède et en particulier chez le canard Barbarie

Dans une approche de recherche de gènes candidats, les profils d'expression d'un nombre restreint de gènes ont été comparés entre les deux lignées BYR et BYN afin de déterminer d'éventuelles différences dans les niveaux d'expression pouvant résulter d'événements génétiques comme des mutations ponctuelles, des délétions, des insertions virales ou des modifications de structure dans la phase codante. Le tableau de la figure 2 présente une liste restreinte de quelques candidats et dont les niveaux d'expression ont été analysés par RT-PCR pour la présente invention dans les yeux des embryons de différentes souches.

### 1.1 Les souches de palmipède utilisées

Les souches utilisées sont des canards correspondant à des animaux de génotypes Pékin à yeux noirs (PN), Barbarie à yeux noirs (BYN) et Barbarie à yeux rouges (BYR). Aucune souche de canard Pékin à yeux rouges n'a été actuellement référencée ou répertoriée. Des échantillons d'hybrides mulards résultants du croisement entre des mâles BYR avec des femelles Pékin YN ont également été utilisés et testés. Dans ces croisements, les phénotypes YR et YN obtenus et observés sont strictement corrélés au sexe. Ainsi, comme résultat de la dominance observée du caractère noir sur le caractère rouge, les mulards mâles obtenus d'un père BYR ont les yeux noirs alors que les femelles ont les yeux rouges. A l'inverse, les mâles Mulards obtenus d'un père BYN ont les yeux noirs comme les femelles qui présentent des yeux noirs.

### 1.2 Prélèvement

Les prélèvements sont réalisés sur le lieu de production des oeufs et des embryons afin d'optimiser la qualité des embryons et des échantillons biologiques. Les yeux sont prélevés par dissection des embryons après lavage dans du PBS (soluté physiologique avec tampon phosphate). Des prélèvements des yeux sont réalisés soit pour l'analyse de l'expression via une détection par réaction de polymérisation en chaîne PCR semi-quantitative (Point II de la présente description) ou quantitative dite aussi PCR en temps réel (point I de la présente description) soit pour l'analyse génomique par PCR génomique (point III de la description). Les embryons Pékin sont prélevés vers 12-13 jours, les embryons mulards vers 14 jours et les embryons Barbarie vers 15-16 jours d'incubation afin d'obtenir des stades de développement comparables. En effet, les durées totales d'incubation sont respectivement de 28, 31 et 35 jours pour les canards Pékin, Mulards et Barbarie.

Pour l'ADN génomique, le sang est prélevé directement au niveau d'une veine de l'aile dans un tube à prélèvement contenant du citrate pour prévenir la coagulation.

Pour chaque série, la moitié des prélèvements a été directement mise dans 0,5 mL de milieu de lyse pour l'acide ribonucléique : RNA (RNA-Bee^{®} commercialisé par Amsbio ou Trizol^{®} commercialisé par Invitrogen) et l'autre moitié dans 0,5 mL de milieu de lyse pour l'obtention d'acide désoxyribonucléique(DNA) décrit ci dessous.

Ainsi pour chaque individu, l'analyse est possible à la fois au niveau expressionnelle et génomique.

### 1.3 Traitement des échantillons

Les prélèvements sont maintenus au froid (4°C) et traités directement par série de 5 échantillons à la fois. L'extraction des RNA est réalisée selon le protocole d'extraction à base de guanidium/isothiocyanate et de phénol avec la solution RNA-Bee^{®} (Amsbio) ou Trizol^{®} (Invitrogen) avec une étape de dissociation du tissu, de déprotéinisation et de précipitation. Une étape de digestion du DNA génomique résiduel a été réalisée par la nucléase DNAse1, suivie d'une purification sur colonne d'affinité Rneasy^{®} (Qiagen) afin d'obtenir la préparation la plus pure possible. La qualité des différents RNA obtenus pour les différents échantillons est satisfaisante comme l'indiquent le dosage réalisé par Nanodrop^{®} avec un ratio des absorbances à 260 nm sur 280 nm compris entre 1,9 et 2,1 et le profil satisfaisant obtenu par dépôt sur gel d'agarose 1% avec la présence classique des bandes 28S et 18S correspondants aux ARNs ribosomaux majoritaires dans les cellules. Les échantillons de RNA sont conservés à -80°C sous la forme d'aliquots. L'ensemble des échantillons a donné des quantités de 15 à 30 µg de RNA total permettant l'approche envisagée de détection de certains gènes candidats.

### 1.4 Dessins des amorces

La connaissance du génome du canard Pékin *Anas platyrhynchos* est récente par la mise ligne d'une première version d'assemblage de ce génome donnée par le site Ensembl (http://pre.ensembl.org/Anas platyynchos/Info/Index). Même si elle est incomplète et ne renseigne que l'espèce Pékin, cette pré-version permet néanmoins de dessiner des oligonucléotides dans le fond génétique canard Pékin, de les tester dans le fond génétique canard Barbarie afin d'identifier l'expression relative des différents gènes candidats de la figure 2 et leur structure génomique dans les deux souches BYN et BYR des canards Barbarie comparativement au canard Pékin de référence.

Le dessin des amorces est réalisé à l'aide du logiciel Primer 3. Afin de déterminer les amorces, les différentes séquences des cDNAs (ADN complémentaires) de l'espèce canard Pékin *Anas platyrhynchos* ont été sélectionnées sur le site Ensembl (figure 1 et SEQ ID NO :1 à 15) et soumises pour le dessin des amorces Sens (S) et Anti-Sens (AS) avec les paramètres préférentiels d'une taille des amorces de 20 nucléotides, d'une taille de l'amplicon de 150 à 250 nucléotides environ, d'un TM de 60°C. Ces différents paramètres sont définis à partir des choix proposés par le site de dessin et selon les recommandations d'une optimisation de la PCR quantitative (Applied). Les séquences des oligonucléotides (voir Tableau 1) ainsi dessinées permettent la synthèse des amorces spécifiques, synthèse réalisée par la suite par la société Eurogentec.

Les amorces convenant pour la détection et la quantification des niveaux d'expression des RNAs sont listées dans le tableau (Tableau 1) ci-dessous :

| | PCR Quantitative | | | |
|---|---|---|---|---|
| Gène étudié | SEQ ID NO | Amorce Sens 5' 3' | SEQ ID NO | Amorce Anti-Sens 5' 3' |
| *ALDH* | 16 | ACTGCCAGAACCACTCAAAGA | 17 | GTGATCCAAGCTCAAAAGCTG |
| *CAT* | 18 | GGTTCTCAACTGTTGCTGGAG | 19 | AAAGACTCAGGGCGAAGACTC |
| *EDN3* | 20 | GCTGAAGCTCAGAAGCAAGAA | 21 | ATATTCCCTGCCAGGTTATGC |
| *EDNRB* | 22 | CTGCACATCATCATCGACATC | 23 | ACTTTGGCACTCCAATTCCTT |
| *GPR143* | 24 | GTCCCTGTCCAGTTGTGAAAA | 25 | TCCGTGTAAATCCCTTGTCTG |
| *LRP6* | 26 | AGAAGCAAACTGCCAAGACAA | 27 | ATGGAGCCTATCGTGTTGTTG |
| *MLPH* | 28 | AGGAAGCTGGATCTCTCCAAG | 29 | TGGGACTGATTTGTCAGGAAC |
| *OCA2* | 30 | CCACTGCTGAAGGTCTCCTC | 31 | ACTTGGCATGAAGGTTGTCC |
| *PAX6* | 32 | CACCACTTCCACAGGTCTCAT | 33 | TGTGTTACAGCATAGGGCACA |
| *RPS17* | 34 | AACGACTTCCACACCAACAAG | 35 | CTCCTCCTCCTGGAGTTTGAT |
| *SLC45A2* | 36 | CCAGAGGGAAAGAGAAGGCTA | 37 | GATCACTGGCTGAACCTACCA |
| *TBP* | 38 | CCAGCTCTTCCACTCACAGAC | 39 | CTCATGATCACAGCAGCAAAA |
| *TBX5* | 40 | AGGATCCCTTCTACAGGTCCA | 41 | AGGTGAAGTGGGCAGAGAAAT |
| *TYR* | 42 | ACACAGGCCCATCCTAGAAGT | 43 | CCTGGAAAGAATCAAGTGCTG |
| *TYRP1* | 44 | TGGAAACACACCACAGTTTGA | 45 | GCAAAGTTCCAGTAGGGAAGG |

### 1.5 Détection de l'expression de différents gènes exprimés dans les yeux embryonnaires

L'analyse de l'expression de 15 gènes dont les deux gènes de ménage RSP17 et TBP est réalisée sur 11 échantillons indépendants prélevés sur des individus différents par PCR quantitative respectivement dans les souches BYN et BYR. Le niveau d'expression est mesuré pour les gènes *ALDH1, CECR2 (CAT), EDN3, EDNRB, GPR143, LRP6, MLPH, OCA2, PAX6, SLC45A2, TBX5, TYR, TYR1* comparativement aux gènes de ménages *RSP17* et *TBP.*

A partir des acides ribonucléiques messagers (mRNAs) extraits dans les différents échantillons (étape 1.3), une réaction de PCR quantitative est réalisée après une étape de réverse transcription.

1 µg de mRNA sont reverse transcrits pendant 1 heure à 50°C dans un mélange contenant 0,5 mM dNTP, 250 ng d'un mélange d'héxanucléotides de séquence aléatoire (Random Primer, Proméga), 4µL de tampon 5X (First Strand Buffer, Invitrogen) composé de 250mM Tris-HCL pH 8.3, 375mM chlorure de potassium, 15mM chlorure de Magnesiumoride, 50mM DTT, 40 unités d'inhibiteur de ribonucléase (RNaseOut®, Invitrogen), 200 unités de reverse transcriptase (Superscript III®,, Invitrogen) et de l'eau exempt de nucléase pour ajuster le volume à 20 µL, selon les indications du fournisseur (Invitrogen).

A l'issue de cette étape, 2 µL de cette réaction diluée au 1/10^{ème} sont utilisés comme matrice pour la réaction de PCR quantitative dans un mélange de 15 µL contenant 7,5 µL d'une solution d'intercalant fluorescent (Mix SyberGreen^{®}, Qiagen^{®} ou Fast Sybr Green Master mix^{®}, Applied), permettant le suivi quantitatif de l'amplicon au cours de la réaction d'amplification, 1 µL d'enzyme Taq équivalent à 1 Unité (MPBio) et 5,35 µL d'H₂O avec les amorces spécifiques susmentionnées dans le tableau 1 de chacun des gènes à 300 nM. La détection a lieu au cours d'une réaction de 40 cycles composés d'une phase de dénaturation à 95°c pendant 30 secondes, d'une phase d'appariement à 55°C pendant 30 secondes et d'une phase d'élongation à 72°C pendant 30 secondes.

Les gènes RSP17 (SEQ ID NO : 10), gène ribosomal, et TBP (SEQ ID NO : 12), gène général de la machinerie de la traduction ont été utilisés comme gène dits « de ménage » ne présentant pas de variations entre les tissus et les échantillons. Ces gènes permettent de quantifier les expressions relatives entre les échantillons et entre les différents tissus. Les réactions de PCR quantitative sont effectuées en plaque de 96 puits dans un appareil MX3000P (Stratagène). Le calcul des expressions relatives est donné par la règle des ΔΔCt dans laquelle Ct est défini comme le seuil de détection de l'espèce amplifiée au cours des réactions de PCR.

### 1.6 Résultat de la PCR quantitative

Le résultat de cette PCR quantitative est illustré sur la figure 3. Cette figure montre ainsi que les 11 individus présentant le phénotype YR (figure 3a) sont globalement homogènes entre eux, de même que les 11 individus présentant le phénotype YN (figure 3b). Il ne varie en effet pas plus de 1 à 2 fois. Par conséquent, il est possible de comparer le ratio YN/YR. La figure 4 est ainsi réalisée. Les données de cette figure indiquent un niveau d'expression du gène *SLC45A2* environ 40 fois plus important dans la souche BYN que dans la souche BYR. Cette différence peut être obtenue avec les couples d'amorces Olig 1S-1AS pour la détection du gène *SLC45A.*

Par conséquent, après avoir testé différents gènes, il apparaît que le gène *SLC45A2* présente un différentiel d'expression important entres les souches BYN et BYR à l'inverse de tous les autres gènes testés. Cette différence est observée quel que soit le gène de référence utilisé, *RSP17* ou *TBP.*

### 1.7 Validation de la PCR quantitative

La validation de l'amplification est faite par le sous clonage des séquences amplifiées par la PCR quantitative dans un vecteur de clonage des fragments de PCR (le vecteur pGEM-Teasy^{®} commercialisé par Promega) et par le séquençage des inserts par la société BioFidal à l'issue du criblage des clones obtenus après transformation bactérienne des produits obtenus après la ligation de ces inserts avec le vecteur pGEML-Teasy ^{®}.

Le séquençage illustré sur la figure 5 montre que les fragments obtenus sont quasiment homologues de la séquence connue de l'espèce canard Pékin mais avec quelques modifications, sans doute propres à l'espèce Barbarie. Une identité de 93% est observée entre les séquences Pékin et Barbarie. On notera une insertion de 15 nucléotides codant pour 5 acides aminés en phase de lecture détectée dans cette séquence du gène *SLC45A2* entre Canard Pékin/Barbarie YR et YN et la séquence prédite de canard Pékin.

Cette approche semble ainsi exclure la possibilité d'une mutation de la phase codante, au moins pour la première partie du gène entre les souches YR et YN. Ainsi, le différentiel d'expression trouve sans doute son origine dans une altération de la régulation et de la transcription du gène liée à une structure génomique altérée du promoteur ou de la région promotrice avec des altérations de certains éléments de régulation de ce gène dans la souche BYR. Par conséquent, la région promotrice du gène va être étudiée par la suite par PCR génomique (partir III de la présente demande).

### II) Profil électrophorétique de l'expression du gène SLC45A2 par PCR semi-quantitative

### 2.1 Dessins des amorces

Le dessin des amorces est réalisé à l'aide du logiciel Primer 3. Afin de déterminer les amorces, la séquence cDNAs (ADN complémentaire) de l'espèce canard Pékin *Anas platyrhynchos du gène SLC45A2* (SEQ. ID NO : 11) a été sélectionnée sur le site Ensembl et soumises pour le dessin des amorces Sens (S) et Anti-Sens (AS) avec les paramètres préférentiels d'une taille des amorces de 20 nucléotides, d'une taille de l'amplicon de 150 à 250 nucléotides environ, d'un TM de 60°C. Ces différents paramètres sont définis à partir des choix proposés par le site de dessin et selon les recommandations d'une optimisation de la PCR quantitative (Applied).

Les séquences des oligonucléotides ainsi dessinées permettent la synthèse des amorces spécifiques, synthèse réalisée par la suite par la société Eurogentec.

Les amorces convenant pour la détection et la quantification des niveaux d'expression des mRNAs pour la PCR semi-quantitative sont les suivantes : Barbarie *Cairana moschata*
Olig 1S: 5' CCAGAGGGAAAGAGAAGGCTA 3' (SEQ ID NO:36)
Olig 1AS: 5' GATCACTGGCTGAACCTACCA 3' (SEQ ID NO:37)
Olig 2S: 5' ATGACCCGCGCTCCATGTCA 3' (SEQ ID NO:46)
Olig-2 AS: 5' CTACCCCACATATCGAACACAA 3' (SEQ ID NO:47)

Ces amorces ont ensuite été testées sur la séquence du cDNA du gène SLC45A2 de la souche BYN qui a été clonée et séquencée par la présente Demanderesse (SEQ ID NO: 48).

### 2.2 Traitement des échantillons

L'analyse de l'expression du gène *SLC45A2* dans les souches BYN et BYR peut être réalisée et visualisée par PCR semi-quantitative à partir des échantillons prélevés sur les embryons (obtenus à l'étape 1.3).

Après une étape préalable de réverse transcription (identique à celle décrite dans le paragraphe 1.5), la réaction de PCR est réalisée dans 20 µL composé de 2 µL de tampon 10x, de 0,4 µL de la solution de dNTP à 0,5 mM, de 250 nM de chaque amorces Sens (Olig 1S et Olig 2S) et Anti-Sens (Olig 1AS et Olig 2AS) et de 1 U d'enzyme Taq (MPBio) selon les recommandations du fabricant.

A l'issue de 35 cycles composés d'une phase de dénaturation à 95°C pendant 30 secondes, d'une phase d'appariement à 55°C pendant 30 secondes et d'une phase d'élongation à 072°C de 30 secondes pour les petits fragments (bandes A et C de la figure 6) à 4 minutes pour les plus grands fragments (bande B et D de la figure 6), les produits d'amplification sont déposés sur gel d'agarose 1%.

### 2.3 Détection du profil électrophorétique résultant de l'amplification par PCR semi-quantitative du niveau relatif d'expression du gène SLC45A2 (figures 6 et 7)

Les fragments ont été amplifiés à l'aide de différents couples d'amorces (Olig 1S- Olig 1AS et Olig 2S -Olig 2AS) comme illustré sur la Figure 6 à partir du matériel génétique de deux individus différents pour la population BYR et BYN. Le profil du canard Pékin (V1) de référence est indiqué.

Les différents appariements comme mentionnés permettent de détecter le fragment amplifié ou son absence relative.

En effet, les deux individus de la souche Barbarie BYR ne présentent pas la bande B à 1556 bp (paire de bases). Par conséquent, il est possible de déterminer à partir d'un échantillon des yeux embryonnaires si un palmipède et notamment un canard présente les yeux rouges ou non, à savoir respectivement s'il y a absence de la bande B à 1556 bp, le canard présentera le phénotype YR et si il y a une bande B, il présentera le phénotype YN. De même, s'il y a absence de bande D, l'individu Barbarie présente le phénotype YR, dans le cas contraire, il est de phénotype YN. Les bandes A et C sont détectables dans les différents individus et attestent de la qualité et de la bonne réalisation de la réaction de PCR avec différents couples d'oligonucléotides.

En utilisant les couples d'oligonucléotides Olig 2S-Olig 2AS et Olig 1S-Olig 2AS, il est ainsi possible de discriminer l'origine de l'échantillon selon son origine YN ou YR par la variation de niveau d'expression. Cette détection peut servir de test à l'identification de ces souches.

La figure 7 illustre des profils électrophorétiques résultant de l'amplification par PCR semi-quantitative du niveau relatif d'expression du gène *SLC45A2* en utilisant le couple d'amorce Olig 2S-Olig 2AS. Les échantillons sont obtenus à partir d'individus de souches Barbarie BYN (A), Barbarie BYR (C) et de mulards obtenus soit de père BYN (B), soit de père BYR (D).

Les individus pour lesquels on peut détecter par amplification une bande à 1556 bp (flèches) dans leur mARN ont un phénotype YN. Ainsi, les individus A2, A8, A11, A15, A19, C2, C1, C7, C3, C10 et D6 présentent le phénotype YN. En effet, A2, A8, A11, A15 et A19 sont des échantillons issus de canards de la souche Barbarie YN et C2, C1, C7, C3 et C10 sont des échantillons provenant de mulards issus de père Barbarie YN et de cane de Pékin YN et qui donc, présentent bien un phénotype YN. Pour l'individu D6, il s'agit d'un mulard issu d'un père Barbarie BYR avec une cane Pékin YN (voir figure 1). Par conséquent, comme cet individu D6 à les YN (la bande est détectée), alors on peut en déduire qu'il s'agit d'un mulard mâle. D10, D7 et D3 sont au contraire des mulards femelles à YR. Leur profil électrophorétique ne présente pas de bande à 1556 bp. De même, B3, B5, B6, B9et B14 sont des individus présentant un phénotype YR car leur profil électrophorétique ne présente pas de bande à 1557 bp. Il s'agit en effet d'échantillons issus de la souche Barbarie BYR.

Ainsi, il apparaît que les souches peuvent être discriminées par l'absence de bande amplifiée à 1556 bp par les amorces Olig 2S-Olig 2AS et Olig 1S-Olig 2AS dans les individus YR. Cette approche peut également identifier les individus mâles et femelles dans les croisements mulards issus d'un père YR (figure 1), mais pas dans les croisements mulards issus d'un père YN.

### III) Profil électrophorétique du gène SLC45A2 par PCR génomique

Pour étudier le profil électrophorétique du gène *SLC45A2* par PCR génomique, on part du même matériel de départ, à savoir les souches de palmipèdes du point 1.1 susmentionné et on utilise la même méthode de prélèvement qui figure au point 1.2.

### 3.1 Traitement des échantillons

Les prélèvements sont maintenus au froid (4°C) et traités directement par série de 5 échantillons à la fois. L'extraction des DNA des échantillons est effectuée selon le protocole SDS Proteinase K. Les échantillons sont directement mis dans le tampon de lyse qui contient 100 mM NaCl, 1 mM EDTA, 10 mM Tris HCl pH 7,5 et 0,2% SDS. La protéinase K (Invitrogen) est ajoutée à la concentration finale de 1 µg/mL et les échantillons incubés pendant 12h à 45°c. De la ribonucléase A (RNAseA, Promega) à 1µg/mL finale est ajoutée pendant 1 heure à 37°C avant l'extraction phénolique par addition de Phénol/Chloroforme/Isoamyl (25 :25 :1 vol/vol) saturé avec 10nM Tris pH 8, 1mM EDTA. La phase aqueuse est précipitée par addition de 2,5 volumes d'éthanol absolu et 0,3M NaAcétate pH 5,3, puis rincée par de l'éthanol 70%. Le dosage et la qualité de l'ADN génomique ainsi extrait sont vérifiés par dosage au Nanodrop^{®} et par dépôt sur gel.

### 3.2 Dessins des amorces

Pour les amorces utilisées pour la détection des fragments génomiques, la séquence génomique du gène *SLC45A2* (ENSAPLG000000003346, SEQ ID NO :49) est obtenue de la pre-version du génome donnée par le site Ensembl (http://pre.ensembl.org/Anas platyrynchos/Info/Index). La séquence est directement copiée dans un fichier et analysée à l'aide du logiciel NTi (Invitrogen) permettant une visualisation de l'ensemble des nucléotides dans ce fragment. Le positionnement théorique des introns et des exons est proposé par Ensembl suite à l'annotation automatique réalisée (figures 7 et 8). Cette carte théorique permet de proposer des amplifications de différents fragments comme indiqué.

Les amorces suivantes ont été trouvées :
Olig-1000 S: 5' TTCAGCAGATAATCACTCTGACGAATTGC 3' (SEQ ID NO:50)
Olig-800 S: 5' ACTTACTTTACAGCACACCCAGGTTTT 3' (SEQ ID NO:51)
Olig-500 S: 5' AAATAATCAGTTTATCGAGGGTGGCACAG 3'
Olig ADN 1S:5' TGTGAGTCCCTCCCCCACTGC 3' (SEQ ID NO:53)
Olig ADN 2AS: 5' CTCGTCCCCATTGAGGTACAAAGCC 3' (SEQ ID NO:54)
Olig ADN 4S: 5' AGGAGCCCTGGGTTACCTGACA 3' (SEQ ID NO:55)
Olig ADN 4AS: 5'ATACTTCCTGGGGTCAGTCACTTCCA 3' (SEQ ID NO:56)
Olig ADN 5S: 5' AGACTCAAAGGCGGATGACCCT 3' (SEQ ID NO:57)
Olig ADN 5 AS: 5'CCTGTCCCATGAAATCCGTGAAGAA 3' (SEQ ID NO:58)
Olig ADN 8 AS: 5'CTACCCCACATATCGAACACAAAAACA 3' (SEQ ID NO:59).

### 3.3 Détection de la structure génomique du locus SLC45A2 chez le canard (figures 8 et 9)

Les ADN génomiques des canards Pékin et des canards Barbarie YN et YR sont analysés au niveau du locus *SLC4522.*

Comme illustré sur la Figure 8 avec le profil obtenu pour deux individus différents, on remarque une absence d'amplification dans les mêmes conditions des fragments qui couvrent la région promotrice. Les couples d'amorces Olig-1000 S- Olig ADN 2AS (absence bande A) et Olig-800 S-Olig ADN 2AS (absence bande B) ne permettent pas d'amplification à partir du matériel génétique de la souche Barbarie BYR contrairement à la souche Barbarie BYN (pistes A et B). Des bouleversements de structure sont donc présents (piste C). Pour la souche Pékin (P), une amplification est également possible et donne un profil similaire à BYN, mais différent par les tailles des amplicons amplifiés (les bandes A à B de canard Pékin n'ont pas la même taille que les bandes A et B de canard BYN). Ainsi, grâce au couple d'amorces Olig-1000 S- Olig ADN 2AS (absence bande A) ou Olig-800 S-Olig ADN 2AS (absence bande B), il est possible de distinguer une population de canard Pékin d'une population de canard Barbarie.

Comme illustré sur la Figure 9, la structure prédite du gène *SLC45A2* donnée par la version de Pre-Ensembl indique la présence de 8 exons codants séparés par des introns de taille variable. Les différents fragments amplifiables entre ces éléments indiquent que la structure du gène est conservée entre les souches BYN et BYR et très similaire au profil obtenu pour le Canard Pékin. En particulier, les pistes A, B et C donnent respectivement des amplifications de 5196 bp environ avec les amorces olig ADN 1S (cf supra) et Olig ADN 4AS, de 4267 bp environ avec les amorces Olig ADN 4S et Olig ADN 5AS et de 5282 bp avec les amorces Olig ADN 5S et Olig ADN 8AS.

La structure du gène n'est donc pas modifiée sensiblement dans les différentes souches et espèces testées. La différence phénotypique n'est donc pas liée à une modification majeure de la structure du locus dans sa partie codante.

Ainsi, les différents couples d'amorces utilisés permettent de conclure à la conservation de la structure globale du gène dans sa partie codante (figure 9), mais à des modifications structurelles importantes dans la région promotrice de ce gène pour la souche BYR. En effet, les produits de la PCR génomique ne sont pas identiques entre les souches YN et YR.

### 3.4 Identification du phénotype et du sexe des animaux par analyse moléculaire génomique du gène SLC45A2 dans des échantillons issus de croisements Mulards (figure 10)

Les échantillons d'ADN génomique obtenus de 16 individus issus du croisement Mulards entre un mâle Barbarie YR et femelle Pékin YN sont analysés par PCR génomique pour détecter la structure du promoteur du gène *SLC45A2* à l'aide des amorces Olig-800 et Olig ADN 2AS qui permet de détecter la présence ou l'absence d'un amplicon de 1790 bp (figure 10A).

En parallèle, le sexe de ces mêmes individus est analysé par la détection de l'amplicon AWS03/UPS3 comme décrit dans Itoh et al., 2011. Cette détection est réalisée à l'aide des oligonucléotides AWS03 S et USP3-CJ AS permettant d'amplifier une bande de 190 nucléotides pour les seuls individus femelles. Le contrôle interne de l'amplification et de la présence du matériel est réalisé par l'amplification d'un fragment de 250 nucléotides du gène Int obtenus avec les oligonucléotides INT-F et INT-R (figure 10B). La séquence des oligonucléotides est celle donnée dans la référence bibliographique Itoh et al., 2011.

La liste des amorces permettant le sexage des animaux est la suivante:
AWS03 S (amorce sens): ACAGTTTGTCTGTCTCCGGGGAA (SEQ ID NO :60);
USP3-CJ AS (amorce anti sens) : AGCTGGACTTCAGTGCATCTTCT (SEQ ID NO :61);
INT-F (amorce sens) : ATAGAAACAATGTGGGAC (SEQ ID NO :62) et,
INT-R(amorce anti-sens) : CTCTGTCTGGAAGGACTT (SEQ ID NO :63).

Ainsi, il apparaît une parfaite corrélation entre la détection de l'amplicon du gène *SLC45A2* de 1500 nucléotide, spécifique des individus YN et l'absence d'amplicon à 190 nucléotides (individus D1, D2, D6, D7, D8, D11, D12 D13 et D15). Ces individus sont mâles.

A l'inverse, l'absence d'amplicon pour le gène *SLC45A2* pour les individus D3, D4, D5, D9, D10, D14, D16, spécifique des individus YR corrèle parfaitement avec la détection de la bande de 190 nucléotides, spécifiques des individus femelles.

Ainsi, par une double PCR sur de l'ADN, il est possible de déterminer le caractère YR et YN des individus testés parfaitement corrélé avec leur sexe.

Bien que l'invention ait été décrite en relation avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

### Références bibliographiques

April CS, Jackson IJ, Kidson SH. (1998). The cloning and sequencing of a cDNA coding for chick tyrosinase-related protein-1. Biochim Biophys Acta. 1395: 7-12.
Bhat SP, Rayner SA, Chau SC, Ariyasu RG. (2004). Pax-6 expression in posthatch chick retina during and recovery from form-deprivation myopia. Dev Neurosci. 26: 328-35.
Chang CM, Coville JL, Coquerelle G, Gourichon D, Oulmouden A, Tixier-Boichard M. (2006). Complete association between a retroviral insertion in the tyrosinase gene and the recessive white mutation in chickens. BMC Genomics. 2006 Feb 5;7:19.
Golz S, Mühleisen T, Schulte D, Mey J. (2008); Regulation of RALDH-1, RALDH-3 and CYP26A1 by transcription factors cVax/Vax2 and Tbx5 in the embryonic chick retina. Int J Dev Neurosci. (5): 435-45.
Gunnarsson U, Hellström AR, Tixier-Boichard M, Minvielle F, Bed'hom B, Ito S, Jensen P, Rattink A, Vereijken A, Andersson L. (2007). Mutations in SLC45A2 cause plumage color variation in chicken and Japanese quail. Genetics. 2007 Feb;175(2):867-77.
Hunter DD, Zhang M, Ferguson JW, Koch M, Brunken WJ. (2004). The extracellular matrix component WIF-1 is expressed during, and can modulate, retinal development. Mol Cell Neurosci. (4): 477-88.
Itoh Y, Suzuki M, Ogawa A, Munechika I, Murata K, Mizuno S. (2001). Identification of the sex of a wide range of Carinatae birds by PCR using primer sets selected from chicken EE0.6 and its related sequences. J Hered. 92: 315-21.
Lopez VM, Decatur CL, Stamer WD, Lynch RM, McKay BS. (2008). L-DOPA is an endogenous ligand for OA1. PLoS Biol. 6: e236.
Mochii M, Mazaki Y, Mizuno N, Hayashi H, Eguchi G. (1998). Role of Mitf in differentiation and transdifferentiation of chicken pigmented epithelial cell. Dev Biol. 193: 47-62.
Nataf V, Lecoin L, Eichmann A, Le Douarin NM. (1996). Endothelin-B receptor is expressed by neural crest cells in the avian embryo. Proc Natl Acad Sci U S A. 93: 9645-50.
Rymer J, Choh V, Bharadwaj S, Padmanabhan V, Modilevsky L, Jovanovich E, Yeh B, Zhang Z, Guan H, Payne W, Wildsoet CF.(2007). The albino chick as a model for studying ocular developmental anomalies, including refractive errors, associated with albinism. Exp Eye Res. 85 : 431-42.
Sturm RA. (2009) Molecular genetics of human pigmentation diversity. Hum Mol Genet. 18(R1):R9-17. Review.
Vaez M, Follett SA, Bed'hom B, Gourichon D, Tixier-Boichard M, Burke T. (2008). A single point-mutation within the melanophilin gene causes the lavender plumage colour dilution phenotype in the chicken. BMC Genet. 9: 7.

## Revendications

1. Procédé d'identification de populations de palmipèdes, ledit procédé étant effectué à partir d'un échantillon biologique prélevé sur l'animal, notamment à partir d'une plume, d'un échantillon de sang ou des fèces, et permettant de vérifier l'appartenance ou la non-appartenance de l'animal sur lequel a été prélevé ledit échantillon biologique à une population de palmipèdes, ce procédé comprend :
- l'amplification de la région promotrice du gène *SLC45A2* dudit échantillon dans un milieu réactionnel comportant au moins un couple d'amorces par une PCR génomique, de sorte à obtenir ou non des produits d'amplifications,
- la séparation des produits d'amplification par électrophorèse sur gel selon leur taille, et
- la comparaison des profils d'électrophorèse obtenus à partir de différentes populations de palmipèdes, telles que des canards Barbarie à Yeux Noirs (BYN), des canards Barbarie à Yeux Rouges (BYR), des canards Pékin à Yeux Noirs (PYN) ou encore des oies, aux fins d'identification de bandes dont le polymorphisme est génétiquement lié au locus du gène *SLC45A2* qui participe à la coloration des yeux et permettant de retrouver, le cas échéant, l'origine de parenté dudit échantillon.

2. Procédé selon la revendication 1, dans lequel le au moins un couple d'amorces 5'-3' utilisé est défini de la manière suivante:
- l'amorce 5' est choisie parmi l'amorce Olig-1000 représentée par la séquence SEQ ID NO :50 suivante : 5' TTCAGCAGATAATCACTCTGACGAATTGC 3' ou toute séquence complémentaire ou dérivée de cette séquence SEQ ID NO : 50, notamment par suppression et/ou substitution et/ou addition d'un ou de plusieurs nucléotides,
- l'amorce 3' est choisie parmi l'amorce Olig ADN 2AS représentée par la séquence SEQ ID NO : 54 suivante : 5' CTCGTCCCCAT1'GAGGTACAAAGCC 3' ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1990 bp sur ledit profil électrophorétique.

3. Procédé selon la revendication 1 ou 2, dans lequel le au moins un couple d'amorces 5'-3' présent dans le milieu réactionnel est également défini de la manière suivante:
- l'amorce 5' est choisie parmi l'amorce Olig-800 représentée par la séquence SEQ ID NO : 51 suivante : 5' ACTTACTTTACAGCACACCCAGGTTTT 3' ou toute séquence complémentaire ou dérivée de cette séquence SEQ ID NO : 51, notamment par suppression et/ou substitution et/ou addition d'un ou de plusieurs nucléotides,
- l'amorce 3' est choisie parmi l'amorce Olig ADN 2AS représentée par la séquence SEQ ID NO : 54 susmentionnée ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1790 bp sur ledit profil électrophorétique.

4. Procédé selon l'une des revendications 2 ou 3, dans lequel le milieu réactionnel comprend un autre couple d'amorces apte à permettre l'identification du sexe dudit palmipède.

5. Procédé selon la revendication 4, dans lequel ledit couple d'amorces apte à permettre l'identification du sexe est défini de la manière suivante :
- l'amorce 5' est choisie parmi l'amorce AWS03 S représentée par la séquence SEQ ID NO : 60 suivante : 5' ACAGTTTGTCTGTCTCCGGGGAA 3' ou toute séquence complémentaire ou dérivée de cette séquence SEQ ID NO : 60, notamment par suppression et/ou substitution et/ou addition d'un ou de plusieurs nucléotides,
- l'amorce 3' est choisie parmi l'amorce USP3 -CJ AS représentée par la séquence SEQ ID NO : 61 suivante : 5'AGCTGGACTTCAGTGCATCTTCT 3' ou toute séquence complémentaire ou dérivée de cette séquence SEQ ID NO : 61, notamment par suppression et/ou substitution et/ou addition d'un ou plusieurs nucléotides.

6. Kit d'identification d'individus ou de populations de palmipèdes par identification de la région promotrice du gène *SLC45A2* mis en oeuvre par le procédé d'identification selon l'une des revendications 1 à 5, comprenant les réactifs appropriés pour l'amplification de la région promotrice du gène *SLC45A2* d'un échantillon environnementale ou animal issu d'une population choisie parmi les palmipèdes, **caractérisé en ce que** le kit comprend :
- un couple d'amorce permettant d'amplifier un fragment de la région promotrice du gène *SLC45A2,*
- des dNTPs et des polymérases.

7. Kit d'identification selon la revendication 6, dans lequel le au moins un couple d'amorces 5'-3' utilisé est défini de la manière suivante :
- l'amorce 5' est choisie parmi l'amorce Olig-1000 représentée par la séquence SEQ ID NO : 50 suivante : 5' TTCAGCAGATAATCACTCTGACGAATTGC 3' ou toute séquence complémentaire ou dérivée de cette séquence SEQ ID NO : 50, notamment par suppression et/ou substitution et/ou addition d'un ou de plusieurs nucléotides,
- l'amorce 3' est choisie parmi l'amorce Olig ADN 2AS représentée par la séquence SEQ ID NO :54 suivante : 5' CTCGTCCCCATTGAGGTACAAAGCC 3' ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1990 bp sur ledit profil électrophorétique.

8. Kit d'identification selon l'une des revendications 6 et 7, dans lequel le kit comprend en outre un autre couple d'amorces défini de la manière suivante:
- l'amorce 5' est choisie parmi l'amorce Olig-800 représentée par la séquence SEQ ID NO : 51 suivante : 5' ACTTACTTTACAGCACACCCAGGTTTT 3' ou toute séquence complémentaire ou dérivée de cette séquence SEQ ID NO : 51, notamment par suppression et/ou substitution et/ou addition d'un ou de plusieurs nucléotides,
- l'amorce 3' est choisie parmi l'amorce Olig ADN 2AS représentée par la séquence SEQ ID NO : 54 susmentionnée ou toute séquence complémentaire ou dérivée de cette séquence SEQ ID NO : 54, notamment par suppression et/ou substitution et/ou addition d'un ou plusieurs nucléotides,
de sorte à obtenir, ou non, une bande de 1790 bp sur ledit profil électrophorétique.

9. Kit d'identification selon l'une des revendications 6 et 8, comprenant un couple d'amorces apte à permettre l'identification du sexe dudit palmipède.

10. Kit d'identification selon la revendication 9, ledit couple d'amorces apte à permettre l'identification du sexe est défini de la manière suivante :
- l'amorce 5' est choisie parmi l'amorce AWS03 S représentée par la séquence SEQ ID NO : 60 suivante : 5' ACAGTTTGTCTGTCTCCGGGGAA 3' ou toute séquence complémentaire ou dérivée de cette séquence SEQ ID NO : 60, notamment par suppression et/ou substitution et/ou addition d'un ou de plusieurs nucléotides,
- l'amorce 3' est choisie parmi l'amorce USP3 -CJ AS représentée par la séquence SEQ ID NO : 61 suivante : AGCTGGACTTCAGTGCATCTTCT ou toute séquence complémentaire ou dérivée de cette séquence SEQ ID NO : 61, notamment par suppression et/ou substitution et/ou addition d'un ou plusieurs nucléotides.

## Patentansprüche

1. Verfahren zur Identifizierung von Palmipedenpopulationen, wobei das Verfahren mit einer dem Tier entnommenen biologischen Probe, vor allem mit einer Feder, einer Blutprobe oder Exkrementen, durchgeführt wird und erlaubt, die Zugehörigkeit oder die Nichtzugehörigkeit des Tiers, dem die biologische Probe entnommen wurde, zu einer Palmipedenpopulation zu überprüfen, wobei dieses Verfahren umfasst:
- das Amplifizieren der Promoterregion des Gens *SLC45A2* der Probe in einem Reaktionsmedium, aufweisend mindestens ein Primerpaar, mittels einer Genom-PCR, um Amplifikationsprodukte zu erhalten oder nicht,
- das Trennen der Amplifikationsprodukte durch Elektrophorese auf Gel gemäß ihrer Größe, und
- das Vergleichen der aus verschiedenen Palmipedenpopulationen wie den Schwarzäugigen Barbarie-Enten (BYN), den Rotäugigen Barbarie-Enten (BYR), den Schwarzäugigen Peking-Enten (PYN) oder auch den Gänsen erhaltenen Elektrophoreseprofile zum Zweck der Identifizierung von Banden, deren Polymorphismus mit dem Locus des Gens *SLC45A2* genetisch verbunden ist, das an der Färbung der Augen beteiligt ist und erlaubt, gegebenenfalls den verwandtschaftlichen Ursprung der Probe festzustellen.

2. Verfahren nach Anspruch 1, wobei das mindestens eine verwendete Primerpaar 5'-3' folgendermaßen definiert ist:
- der Primer 5' ist aus dem Primer Olig-1000 ausgewählt, dargestellt durch die folgende Sequenz SEQ ID NO : 50: 5' TTCAGCAGATAATCACTCTGACGAATTGC 3' oder jede komplementäre oder von dieser Sequenz SEQ ID NO : 50 abgeleitete Sequenz, insbesondere durch Suppression und/oder Substitution und/oder Addition eines oder mehrerer Nukleotide,
- der Primer 3' ist aus dem Primer Olig ADN 2AS ausgewählt, dargestellt durch die folgende Sequenz SEQ ID NO : 54: 5' CTCGTCCCCATTGAGGTACAAAGCC 3' oder jede komplementäre oder von dieser Sequenz SEQ ID NO : 54 abgeleitete Sequenz, insbesondere durch Suppression und/oder Substitution und/oder Addition eines oder mehrerer Nukleotide,
um ein Band von 1990 bp auf dem Elektrophoreseprofil zu erhalten oder nicht.

3. Verfahren nach Anspruch 1 oder 2, wobei das mindestens eine Primerpaar 5'-3', das in dem Reaktionsmedium vorhanden ist, ebenfalls folgendermaßen definiert ist:
- der Primer 5' ist aus dem Primer Olig-800 ausgewählt, dargestellt durch die folgende Sequenz SEQ ID NO : 51: 5' ACTTACTTTACAGCACACCCAGGTTTT 3' oder jede komplementäre oder von dieser Sequenz SEQ ID NO : 51 abgeleitete Sequenz, insbesondere durch Suppression und/oder Substitution und/oder Addition eines oder mehrerer Nukleotide,
- der Primer 3' ist aus dem Primer Olig ADN 2AS ausgewählt, dargestellt durch die obigen Sequenz SEQ ID NO : 54 oder jede komplementäre oder von dieser Sequenz SEQ ID NO : 54 abgeleitete Sequenz, insbesondere durch Suppression und/oder Substitution und/oder Addition eines oder mehrerer Nukleotide,
um ein Band von 1790 bp auf dem Elektrophoreseprofil zu erhalten oder nicht.

4. Verfahren nach einem der Ansprüche 2 oder 3, wobei das Reaktionsmedium ein anderes Primerpaar umfasst, das imstande ist, die Identifizierung des Geschlechts des Palmipeden zu erlauben.

5. Verfahren nach Anspruch 4, wobei das Primerpaar, das imstande ist, die Identifizierung des Geschlechts zu erlauben, folgendermaßen definiert ist:
- der Primer 5' ist aus dem Primer AWS03 S ausgewählt, dargestellt durch die folgende Sequenz SEQ ID NO : 60: 5' ACAGTTTGTCTGTCTCCGGGGAA 3' oder jede komplementäre oder von dieser Sequenz SEQ ID NO : 60 abgeleitete Sequenz, insbesondere durch Suppression und/oder Substitution und/oder Addition eines oder mehrerer Nukleotide,
- der Primer 3' ist aus dem Primer USP3 -CJ AS ausgewählt, dargestellt durch die folgende Sequenz SEQ ID NO : 61: 5'AGCTGGACTTCAGTGCATCTTCT 3' oder jede komplementäre oder von dieser Sequenz SEQ ID NO : 61 abgeleitete Sequenz, insbesondere durch Suppression und/oder Substitution und/oder Addition eines oder mehrerer Nukleotide.

6. Identifikationskit von Individuen oder Populationen von Palmipeden durch Identifizierung der Promoterregion des Gens *SLC45A2,* umgesetzt mittels des Identifizierungsverfahrens nach einem der Ansprüche 1 bis 5, umfassend Reagenzien, die für die Amplifizierung der Promoterregion des Gens *SLC45A2* einer Umwelt- oder Tierprobe einer Population, ausgewählt aus den Palmipeden, geeignet sind, **dadurch gekennzeichnet, dass** das Kit umfasst:
- ein Primerpaar, das erlaubt, ein Fragment der Promoterregion des Gens *SLC45A2* zu amplifizieren,
- dNTPs und Polymerasen.

7. Identifikationskit nach Anspruch 6, wobei das mindestens eine verwendete Primerpaar 5'-3' folgendermaßen definiert ist:
- der Primer 5' ist aus dem Primer Olig-1000 ausgewählt, dargestellt durch die folgende Sequenz SEQ ID NO : 50: 5' TTCAGCAGATAATCACTCTGACGAATTGC 3' oder jede komplementäre oder von dieser Sequenz abgeleitete Sequenz SEQ ID NO : 50, insbesondere durch Suppression und/oder Substitution und/oder Addition eines oder mehrerer Nukleotide,
- der Primer 3' ist aus dem Primer Olig ADN 2AS ausgewählt, dargestellt durch die folgende Sequenz SEQ ID NO : 54: 5' CTCGTCCCCATTGAGGTACAAAGCC 3' oder jede komplementäre oder von dieser Sequenz SEQ ID NO : 54 abgeleitete Sequenz, insbesondere durch Suppression und/oder Substitution und/oder Addition eines oder mehrerer Nukleotide,
um ein Band von 1990 bp auf dem Elektrophoreseprofil zu erhalten oder nicht.

8. Identifikationskit nach einem der Ansprüche 6 und 7, wobei das Kit ferner ein anderes Primerpaar umfasst, das folgendermaßen definiert ist:
- der Primer 5' ist aus dem Primer Olig-800 ausgewählt, dargestellt durch die folgende Sequenz SEQ ID NO : 51: 5' ACTTACTTTACAGCACACCCAGGTTTT 3' oder jede komplementäre oder von dieser Sequenz SEQ ID NO : 51 abgeleitete Sequenz, insbesondere durch Suppression und/oder Substitution und/oder Addition eines oder mehrerer Nukleotide,
- der Primer 3' ist aus dem Primer Olig ADN 2AS ausgewählt, dargestellt durch die obige Sequenz SEQ ID NO : 54 oder jede komplementäre oder von dieser Sequenz SEQ ID NO : 54 abgeleitete Sequenz, insbesondere durch Suppression und/oder Substitution und/oder Addition eines oder mehrerer Nukleotide,
um ein Band von 1790 bp auf dem Elektrophoreseprofil zu erhalten oder nicht.

9. Identifikationskit nach einem der Ansprüche 6 und 8, umfassend ein Primerpaar, das imstande ist, die Identifizierung des Geschlechts des Palmipeden zu erlauben.

10. Identifikationskit nach Anspruch 9, wobei das Primerpaar, das imstande ist, die Identifizierung des Geschlechts zu erlauben, folgendermaßen definiert ist:
- der Primer 5' ist aus dem Primer AWS03 S ausgewählt, dargestellt durch die folgende Sequenz SEQ ID NO : 60: 5' ACAGTTTGTCTGTCTCCGGGGAA 3' oder jede komplementäre oder von dieser Sequenz SEQ ID NO : 60 abgeleitete Sequenz, insbesondere durch Suppression und/oder Substitution und/oder Addition eines oder mehrerer Nukleotide,
- der Primer 3' ist aus dem Primer USP3 -CJ AS ausgewählt, dargestellt durch die folgende Sequenz SEQ ID NO : 61: AGCTGGACTTCAGTGCATCTTCT oder jede komplementäre oder von dieser Sequenz SEQ ID NO : 61 abgeleitete Sequenz, insbesondere durch Suppression und/oder Substitution und/oder Addition eines oder mehrerer Nukleotide.

## Claims

1. A method for identifying populations of palmipeds, said method being carried out from a biological sample taken on the animal, notably from a feather, a blood sample or faeces, and giving the possibility of verifying whether the animal on which was taken said biological sample belongs or not to a population of palmipeds, this method comprises:
- the identification of the promoter region of the gene SLC45A2 of said sample in a reaction medium including at least a pair of primers by genomic PCR, so as to either obtain all not amplification products,
- the separation of the amplification products by electrophoresis on a gel according to their size, and
- the comparison of the electrophoresis profiles obtained from different populations of palmipeds, such as Black-Eyed Muscovy ducks (BYN), Red-Eyed Muscovy ducks (BYR), Black-Eyed Pekin ducks (PYN) or further geese with the purpose of identifying bands, the polymorphism of which is genetically related to the locus of the SLC45A2 gene which participates in the coloration of the eyes and giving the possibility of finding where appropriate the original relationship of said sample.

2. The method according to claim 1, wherein said at least one pair of primers 5'-3' used is defined in the following way:
- the primer 5' is selected from the primer Olig-1000 illustrated by the following sequence SEQ ID NO:50: 5' TTCAGCCAGAATAATCACTCTGACGAATTGC 3' or any complementary sequence or derived from this sequence SEQ ID NO:50, notably by suppression and/or substitution and/or addition of one or of several nucleotides,
- the primer 3' is selected from among the primer Olig DNA 2AS represented by the following sequence SEQ ID NO:54: 5' CTCGTCCCCATTGAGGTACAAAGCC 3' or any complementary sequence or derived from this sequence SEQ ID NO:54, notably by suppression and/or substitution and/or addition of one or of several nucleotides,
so as to obtain, or not, a band of 1990 bp on said electrophoretic profile.

3. The method according to claim 1 or 2, wherein said at least one pair of primers 5'-3' present in the reaction medium is also defined in the following way:
- the primer 5' is selected from the primer Olig-800 illustrated by the following sequence SEQ ID NO:51: 5' ACTTACTTTACAGCACACCCAGGTTTT 3' or any complementary sequence or derived from this sequence SEQ ID NO:51, notably by suppression and/or substitution and/or addition of one or of several nucleotides,
- the primer 3' is selected from among the primer Olig DNA 2AS represented by the aforementioned sequence SEQ ID NO:54: or any complementary sequence or derived from this sequence SEQ ID NO:54, notably by suppression and/or substitution and/or addition of one or of several nucleotides,
so as to obtain, all, a band of 1790 bp on said electrophoretic profile.

4. The method according to one of claims 2 or 3, wherein the reaction medium comprises another pair of primers are capable of giving the possibility of identifying the gender of said palmiped.

5. The method according to claim 4, wherein said pair of primers capable of giving the possibility of identifying the gender is defined in the following way:
- the primer 5' is selected from among the primer AWS03 S illustrated by the following sequence SEQ ID NO:60: 5' ACAGTTTGTCTGTCTCCGGGGAA 3' or any complementary sequence or derived from this sequence SEQ ID NO:60, notably by suppression and/or substitution and/or addition of one or of several nucleotides,
- the primer 3' is selected from among the primer USP3 -CJ AS represented by the following sequence SEQ ID NO:61: 5 'AGCTGGACTTCAGTGCATCTTCT 3' or any complementary sequence or derived from this sequence SEQ ID NO:61, notably by suppression and/or substitution and/or addition of one or of several nucleotides.

6. An identification kit for individuals or populations of palmipeds by identification of the promoter region of the gene SLC45A2 applied by the identification method according to one of claims 1 to 5, comprising suitable reagents for amplifying the promoter region of the gene SLC45A2 of an environmental or animal sample from a population selected from among palmipeds, characterized that the kit comprises:
- a pair of primers giving the possibility of amplifying a fragment of the promoter region of the gene SLC45A2,
- dNTPs and polymerases.

7. The identification kit according to claim 6, wherein said at least one pair of primers 3'-5' used is defined in the following way:
- the primer 5' is selected from the primer Olig-1000 illustrated by the following sequence SEQ ID NO:50: 5' TTCAGCCAGAATAATCACTCTGACGAATTGC 3' or any complementary sequence or derived from this sequence SEQ ID NO:50, notably by suppression and/or substitution and/or addition of one or of several nucleotides,
- the primer 3' is selected from among the primer Olig DNA 2AS represented by the following sequence SEQ ID NO:54: 5' CTCGTCCCCATTGAGGTACAAAGCC 3' or any complementary sequence or derived from this sequence SEQ ID NO:54, notably by suppression and/or substitution and/or addition of one or of several nucleotides,
so as to obtain, or not, a band of 1990 bp on said electrophoretic profile.

8. The identification kit according to one of claims 6 and 7, wherein the kit further comprises another pair of primers are defined in the following way:
- the primer 5' is selected from the primer Olig-800 illustrated by the following sequence SEQ ID NO:51: 5' ACTTACTTTACAGCACACCCAGGTTTT 3' or any complementary sequence or derived from this sequence SEQ ID NO:51, notably by suppression and/or substitution and/or addition of one or of several nucleotides,
- the primer 3' is selected from among the primer Olig DNA 2AS represented by the aforementioned sequence SEQ ID NO:54: or any complementary sequence or derived from this sequence SEQ ID NO:54, notably by suppression and/or substitution and/or addition of one or of several nucleotides,
so as to obtain, or not, a band of 1790 bp on said electrophoretic profile.

9. The identification kit according to one of claims 6 and 8, comprising a pair of primers capable of giving the possibility of identifying the gender of said palmiped.

10. The identification kit according to claim 9, said pair of primers capable of giving the possibility of identifying the gender is defined in the following way:
- the primer 5' is selected from among the primer AWS03 S illustrated by the following sequence SEQ ID NO:60: 5' ACAGTTTGTCTGTCTCCGGGGAA 3' or any complementary sequence or derived from this sequence SEQ ID NO:60, notably by suppression and/or substitution and/or addition of one or of several nucleotides,
- the primer 3' is selected from among the primer USP3 -CJ AS represented by the following sequence SEQ ID NO:61: AGCTGGACTTCAGTGCATCTTCT or any complementary sequence or derived from this sequence SEQ ID NO:61, notably by suppression and/or substitution and/or addition of one or of several nucleotides.
